# EUROPEAN PATENT APPLICATION

(11) **EP 3 605 089 A1**
(43) Date of publication of application: **05.02.2020**
(21) Application number: 18772241.8
(22) Date of filing: 20.03.2018
(51) Int. Cl.: G01N 33/53

(54) **METHOD FOR FORMING COMPLEX OF SUBSTANCE HAVING SUGAR CHAIN AND LECTIN**

(30) Priority: 22.03.2017 JP 2017055411
(71) Applicant: FUJIFILM Wako Pure Chemical Corporation, Osaka-shi, Osaka 540-8605 (JP)
(72) Inventor: YAMASHITA Kenichiro, Amagasaki-shi Hyogo 661-0963 (JP); ISHIKAWA Tomokazu, Amagasaki-shi Hyogo 661-0963 (JP); KUROSAWA Tatsuo, Amagasaki-shi Hyogo 661-0963 (JP)
(74) Representative: Winter, Brandl, Fürniss, Hübner, Röss, Kaiser, Polte - Partnerschaft mbB
(86) International application number: PCT/JP2018/010966
(87) International publication number: WO 2018/174044

(57) **Abstract**

An object of the present invention is to provide a method for increasing an amount of a complex of a substance having a sugar chain and a lectin.

The present invention relates to "a method for forming a complex of a substance having a sugar chain and a lectin having affinity with the sugar chain of the substance having a sugar chain, the method including: bringing a sample containing the substance having a sugar chain into contact with the lectin in the presence of a water-soluble polysaccharide having no N-acetylglucosamine or a water-soluble compound having a polysaccharide having no N-acetylglucosamine (polysaccharides according to the present invention)" and to a "enhancer for forming a complex of a substance having a sugar chain and a lectin having affinity with the sugar chain of the substance having a sugar chain, the enhancer including: the polysaccharides according to the present invention".

## Description

### Technical Field

The present invention relates to a method for forming a complex of a substance having a sugar chain and a lectin.

### Background Art

A sugar chain is a compound obtained such that the same kinds or two or more kinds of monosaccharide molecules bond to each other through a glycoside bond.

Sugar chains are mainly present on a cell surface in a form of glycoconjugate bonding to a protein or lipid in a living body. Sugar chains are involved in important physiological actions in a living body, such as cell proliferation, bacterial or viral infection, nerve elongation, inflammation, and immunization.

The kind and number of sugar chains added to a substance having a sugar chain of a glycoprotein or the like present in a living body are usually constant. However, in some cases, a sugar chain changes in a disease-specific manner. Therefore, such a sugar chain is used as a biomarker for diagnosis.

For example, a sugar chain or a glycoprotein of which a sugar chain changes accompanied by cell canceration and cancer progression is known as a tumor marker.

α-Fetoprotein (AFP: liver cancer marker), a prostate specific antigen (PSA: prostate cancer marker), a carcinoembryonic antigen (CEA: colorectal cancer marker), carbohydrate antigen 19-9 (CA19-9: pancreatic cancer marker, gastrointestinal cancer marker), and podocalyxin are known as tumor markers having a sugar chain.

A method in which a lectin is used is generally used as a method for detecting and analyzing a sugar chain. A lectin is a substance having properties of specifically recognizing a sugar chain and bonding thereto among proteins or glycoproteins present in plants, animals, microorganisms, and the like, and is a generic term for those excluding enzymes and antibodies. Since a lectin has high specificity to a sugar chain, it is possible to specifically detect a substance having a sugar chain using a lectin.

A surface plasmon resonance method, a lectin electrophoresis method (for example, capillary electrophoresis), a lectin column method (for example, lectin affinity chromatography), a lectin microarray method, and an immunological measurement method such as a sandwich method in which a lectin immobilized on an insoluble carrier or a biotin-labeled lectin is used are known as the method for detecting a sugar chain or a substance having a sugar chain using a lectin.

### Citation List

### Patent Literature

Patent Literature 1 Japanese Patent No. 4862093

### Summary of Invention

### Technical Problem

Although a lectin has high specificity in recognition of a sugar chain, it is known that a bonding force of a lectin to a sugar chain is extremely weaker than that of an antibody to a sugar chain. For example, a dissociation constant of an antigen-antibody reaction is about 10⁻⁹ to 10⁻⁷, but a dissociation constant between a lectin and a sugar chain is only about 10⁻⁷ to 10⁻³ (Jun Hirabayashi, et al, Chem. Soc. Rev., Vol. 442, pp. 4443-4458, 2013, and the like). For this reason, it is difficult to form a firm complex using a lectin and a sugar chain unlike in the antigen-antibody reaction. Therefore, in a case where a measurement method for forming a complex of a lectin and a substance having a sugar chain and measuring the amount of complex thereof to measure the substance having a sugar chain is carried out, the amount of complex obtained is insufficient. Therefore, there is a problem in that satisfactory measurement sensitivity is not obtained.

It should be noted that Japanese Patent No. 4862093 (Patent Literature 1) discloses a method for forming a complex of an affinity molecule having an affinity with an analyte such as an antibody or a lectin and a charge carrier molecule in the presence of polyanionic polymer. However, in the literature, an antigen-antibody reaction is performed in the presence of a polyanionic polymer, but there is no specific disclosure about a reaction between a substance having a sugar chain and a lectin. For this reason, it is unclear from the disclosure of Patent Literature 1 whether or not the polyanionic polymer has any influence on an interaction between a sugar chain and a lectin.

From the above, an object of the present invention is to provide a method for increasing an amount of complex of a substance having a sugar chain and a lectin.

### Solution to Problem

The present inventors conducted extensive studies to solve the problem. As a result, they have found that it is possible to solve the problem by increasing the amount of complex of a substance having a sugar chain and a lectin by performing a reaction for forming a complex of a sugar chain and a lectin in the presence of a water-soluble polysaccharide having no N-acetylglucosamine or a water-soluble compound having a polysaccharide having no N-acetylglucosamine, and have completed the present invention.

The present invention is configured as follows.
(1) A method for forming a complex of a substance having a sugar chain and a lectin having affinity with the sugar chain of the substance having a sugar chain, the method comprising: bringing a sample containing the substance having a sugar chain into contact with the lectin in the presence of a water-soluble polysaccharide having no N-acetylglucosamine or a water-soluble compound having a polysaccharide having no N-acetylglucosamine.
(2) The method according to (1), in which the water-soluble polysaccharide having no N-acetylglucosamine or the water-soluble compound having a polysaccharide having no N-acetylglucosamine does not have a sugar chain with which the lectin has affinity.
(3) The method according to (1) or (2), in which the water-soluble polysaccharide having no N-acetylglucosamine or the water-soluble compound having a polysaccharide having no N-acetylglucosamine is dextran sulfate or a salt thereof, or chondroitin sulfate c or a salt thereof.
(4) The method according to any one of (1) to (3), which is selected from (i) and (ii), in which (i) the water-soluble polysaccharide having no N-acetylglucosamine is dextran sulfate or a salt thereof, the substance having a sugar chain is α-fetoprotein-L3 (AFP-L3), and the lectin is a lens culinaris agglutinin, and (ii) the water-soluble polysaccharide having no N-acetylglucosamine or the water-soluble compound having a polysaccharide having no N-acetylglucosamine is dextran sulfate or a salt thereof, or chondroitin sulfate c or a salt thereof, the substance having a sugar chain is an α(2,3)-sugar chain free type prostate specific antigen, and the lectin is Maackia amurensis lectin.
(5) An enhancer for forming a complex of a substance having a sugar chain and a lectin having affinity with the sugar chain of the substance having a sugar chain, the enhancer comprising: a water-soluble polysaccharide having no N-acetylglucosamine or a water-soluble compound having a polysaccharide having no N-acetylglucosamine.
(6) The enhancer for forming a complex according to (5), in which the water-soluble polysaccharide having no N-acetylglucosamine or the water-soluble compound having a polysaccharide having no N-acetylglucosamine does not have a sugar chain with which the lectin has affinity.
(7) The enhancer for forming a complex according to (5) or (9)6, in which the water-soluble polysaccharide having no N-acetylglucosamine or the water-soluble compound having a polysaccharide having no N-acetylglucosamine is dextran sulfate or a salt thereof, or chondroitin sulfate c or a salt thereof.
(8) The enhancer for forming a complex according to any one of (5) to (7) which is selected from the following (i) and (ii): in which (i) the water-soluble polysaccharide having no N-acetylglucosamine is dextran sulfate or a salt thereof, the substance having a sugar chain is α-fetoprotein-L3 (AFP-L3), and the lectin is a lens culinaris agglutinin, and (ii) the water-soluble polysaccharide having no N-acetylglucosamine or the water-soluble compound having a polysaccharide having no N-acetylglucosamine is dextran sulfate or a salt thereof, or chondroitin sulfate c or a salt thereof, the substance having a sugar chain is an α(2,3) sugar chain free type prostate specific antigen, and the lectin is Maackia amurensis lectin.
(9) A method for measuring a substance having a sugar chain, the method comprising: forming the complex of the substance having a sugar chain and the lectin in the method according to (1); and measuring an amount of the complex.

### Advantageous Effects of Invention

The amount of complex of a substance having a sugar chain and a lectin is increased by carrying out the method for forming a complex of the present invention. For this reason, it is possible to perform highly sensitive measurement of the substance having a sugar chain using the method for forming a complex of the present invention as long as the substance having a sugar chain is measured in the presence of a water-soluble polysaccharide having no N-acetylglucosamine or a water-soluble compound having a polysaccharide having no N-acetylglucosamine.

In addition, the method for forming a complex of the present invention can be used for any reaction, detection, measurement, analysis of a sugar chain, and the like in which affinity of a lectin with a sugar chain is used. There are effects in that the sensitivity of detection or measurement can be enhanced, the analysis of a sugar chain can be performed with high accuracy, and a substance having a sugar chain can be efficiently separated from a lectin.

### (Brief Description of Drawings)

Fig. 1 shows a sensorgram (+) obtained through measurement with Biacore using an AFP-L3 solution containing sodium dextran sulfate, and a sensorgram (◆) obtained through measurement with Biacore using an AFP-L3 solution containing no sodium dextran sulfate, which were obtained in Example 1.
Fig. 2 shows a sensorgram (□) obtained through measurement with Biacore using an AFP-L3 solution containing sodium chondroitin sulfate c, and a sensorgram (◆) obtained through measurement with Biacore using an AFP-L3 solution containing no sodium chondroitin sulfate c, which were obtained in Example 1.
Fig. 3 is a schematic diagram of a microchip used in Example 2 and Example 3.
Fig. 4 is a schematic diagram of a flow path of the microchip used in Example 2 and Example 3.
Fig. 5 is a graph showing a relationship, obtained in Example 2, between a concentration of sodium chondroitin sulfate c and a peak area of a fraction of a complex 1.
Fig. 6 is a graph showing a relationship, obtained in Example 3, between a concentration of sodium dextran sulfate and a peak area of a fraction of a complex 1.

### Description of Embodiments

### [1] Method for Forming Complex of Present Invention

The method for forming a complex according to the embodiment of the present invention is a "method for forming a complex of a substance having a sugar chain and a lectin having affinity with the sugar chain of the substance having a sugar chain, in which a sample containing the substance having a sugar chain is brought into contact with the lectin in the presence of a water-soluble polysaccharide having no N-acetylglucosamine or a water-soluble compound having a polysaccharide having no N-acetylglucosamine".

In some cases, the "lectin having affinity with a sugar chain of a substance having a sugar chain" is simply described as a "lectin" below.

### Water-Soluble Polysaccharide Having no N-Acetylglucosamine

The water-soluble polysaccharide having no N-acetylglucosamine according to the present invention is usually called a polysaccharide, and an example thereof includes a water-soluble polysaccharide having no N-acetylglucosamine as constituent sugar.

Examples thereof includes water-soluble polysaccharides, such as a homopolysaccharide (simple polysaccharide) in which one kind of monosaccharide is polymerized many times through a glycoside bond, a heteropolysaccharide (conjugated polysaccharide) in which plural kinds of monosaccharides are polymerized many times through a glycoside bond, or sugar alcohol, which have no N-acetylglucosamine as constituent sugar.

Examples of a water-soluble homopolysaccharide (simple polysaccharide) having no N-acetylglucosamine as constituent sugar include dextran, agarose, carrageenan, dextran sulfate, carboxymethyl dextran, fucoidan, funoran, chitosan, and derivatives thereof.

Examples of a heteropolysaccharide (conjugated polysaccharide) having no N-acetylglucosamine as constituent sugar include porphyran, glucomannan, alginic acid, xanthan gum, and derivatives thereof.

Examples of water-soluble sugar alcohol having no N-acetylglucosamine as constituent sugar include erythritol, xylitol, and derivatives thereof.

Among these water-soluble polysaccharides having no N-acetylglucosamine, the water-soluble homopolysaccharide is preferable, the dextran sulfate and a derivative thereof are more preferable, and the dextran sulfate is particularly preferable.

The water-soluble polysaccharides having no N-acetylglucosamine according to the present invention may be, for example, alkali metal salts such as lithium salt, sodium salt, and potassium salt, alkaline earth metal salts such as calcium salt and magnesium salt, and organic amine salts such as ammonium salt, triethylamine salt, and dimethylamine salt.

The preferred salts vary depending on the kind of polysaccharide or the kind of compound having a polysaccharide, but the sodium salt is preferable and the sodium dextran sulfate is more preferable.

In addition, a molecular weight of the water-soluble polysaccharide having no N-acetylglucosamine according to the present invention is several hundreds of to several millions, preferably about 1,000 to 1,000,000, and more preferably about 5,000 to 500,000.

### Water-Soluble Compound Having Polysaccharide Having no N-Acetylglucosamine

An example of the water-soluble compound having a polysaccharide having no N-acetylglucosamine according to the present invention includes so-called water-soluble glycoconjugate in which a protein or lipid bonds to a polysaccharide and which does not have N-acetylglucosamine as constituent sugar of the polysaccharide moiety.

An example thereof includes glycoconjugate such as proteoglycan, in which a protein bonds to glycosaminoglycan, and which does not have N-acetylglucosamine as constituent sugar of the glycosaminoglycan moiety.

Examples of such water-soluble proteoglycan include chondroitin sulfate such as chondroitin sulfate a, chondroitin sulfate b, chondroitin sulfate c, chondroitin sulfate d, chondroitin sulfate e, and derivatives thereof. Among these, the chondroitin sulfate c and a derivative thereof are preferable and the chondroitin sulfate c is particularly preferable.

The water-soluble compound having a polysaccharide having no N-acetylglucosamine according to the present invention may be, for example, alkali metal salts such as lithium salt, sodium salt, and potassium salt, alkaline earth metal salts such as calcium salt and magnesium salt, and organic amine salts such as ammonium salt, triethylamine salt, and dimethylamine salt.

The preferred salts vary depending on the kind of polysaccharide or the kind of compound having a polysaccharide, but the sodium salt is preferable. For example, sodium chondroitin sulfate is preferable and sodium chondroitin sulfate c is more preferable.

In addition, a molecular weight of the water-soluble compound having a polysaccharide having no N-acetylglucosamine according to the present invention is several hundreds of to several millions, preferably about 1,000 to 1,000,000, and more preferably about 5,000 to 500,000.

One showing sufficient water solubility to a degree of entering a homogeneously dissolved state in a reaction solution under the conditions during a reaction in a case where a substance having a sugar chain and a lectin are brought into contact with each other for the reaction in the presence of the water-soluble polysaccharide having no N-acetylglucosamine or the water-soluble compound having a polysaccharide having no N-acetylglucosamine according to the present invention is selected as the water-soluble polysaccharide having no N-acetylglucosamine or the water-soluble compound having a polysaccharide having no N-acetylglucosamine according to the present invention. The preferred specific examples thereof are as described above.

It should be noted that, in some cases, the "water-soluble polysaccharide having no N-acetylglucosamine or the water-soluble compound having a polysaccharide having no N-acetylglucosamine" is collectively described as "polysaccharides according to the present invention" below.

### Substance Having Sugar Chain

The "substance having a sugar chain" according to the embodiment of the present invention is not particularly limited as long as it is a substance having a "sugar chain having a sugar chain structure with which a lectin has affinity (sugar chain structure to which a lectin bonds)".

Specific examples of the substance having a sugar chain include tumor markers such as AFP, PSA, CA19-9, CA125, PIVKA-II, and podocalyxin, serum proteins such as α1-globulin, α2-globulin, β-globulin, γ-globulin, and fetuin, fiber proteins such as collagen, hormones such as gonadotropin, a thyroid-stimulating hormone, human chorionic gonadotropin (hCG), a luteinizing hormone (LH), a follicle-stimulating hormone (FSH), and a thyroid-stimulating hormone (TSH), enzymes such as ribonuclease, Taka-amylase, γ-glutamyl transferase (γ-GTP), alkaline phosphatase, cholinesterase, and lysozyme, intercellular substances such as fibronectin and proteoglycan, an α1-acidic glycoprotein, α1-antitrypsin, α2-macroglobulin, an α2-HS glycoprotein, transferrin, haptoglobin, ceruloplasmin, a carcinoembryonic antigen (CEA), immunoglobulin (IgG, IgM, IgA, IgD, IgE), complement components (C1, C1q, C1r, C1s, C4, C2, C3, C5, C6, C7, C8, C9), interferon, blood coagulation factors (such as fibrinogen, prothrombin, a V-factor, a VII-factor, a IX-factor, a X-factor, a XI-factor, a XII-factor, and a XIII factor), antithrombin III, erythropoietin, and undifferentiation markers (such as SSEQ-1, SSEA-3/4, and podocalyxin) of pluripotent stem cells (such as ES cells and iPS cells).

As the substance having a sugar chain according to the embodiment of the present invention, the AFP, the PSA, and the podocalyxin are preferable and the AFP and the PSA are more preferable.

The substance having a sugar chain according to the embodiment of the present invention also includes a "sugar chain" having a sugar chain structure with which a lectin has affinity. Examples thereof include amylose, amylopectin, cellulose, and glycogen. Lewis X (Galβ1-4[fucα1-3]GlcNac) whose expression has been confirmed in undifferentiated stem cells such as mouse ES cells or a fragment of a sugar chain which is released from a glycoprotein or the like are also contained in the substance having a sugar chain according to the embodiment of the present invention. A preferred example includes Lewis X (Galβ1-4[fucα1-3]GlcNac).

An example of the sugar chain relating to the "substance having a sugar chain" according to the embodiment of the present invention includes a sugar chain having a sugar chain structure with which a lectin has affinity.

Examples thereof include the sugar chains included in the substance having a sugar chain. In addition, an example thereof includes a sugar chain observed in the blood or the like of a cancer patient.

Specific examples thereof include:
(1) a sugar chain to which fucose is added; for example, Fucα1-6GlcNac (core fucose) (Kobayashi Y. et al., J. Biol. Chem., Vol. 287, No. 41, pp. 33973-33982, October 5, 2012) which is a sugar chain of APF-L3 observed in a patent with liver cancer;
(2) a sugar chain to which sialic acid is added at a terminal, for example, Siaα2-3Gal (Chikara Oyama et al., Glycobiology, Vol. 14, No. 8, pp. 671-679, 2004, Yoneyama T. et al., Biochem. Biophys. Res. Commun., Vol. 448, No. 4, pp. 390-396, 2014, Tomokazu Ishikawa et al., J. Urology, Vol. 196, p. e331) which is a sugar chain of PSA observed in a patent with liver cancer; and
(3) a sugar chain to which N-acetylgalactosamine is added; for example, GalNacβ1-R (Japanese Patent No. 5630757, Takatoshi Kaya, et al., Anal. Chem., Vol. 87, No. 3, pp. 1797-1803, 2015) which is a sugar chain of PSA observed in a patent with liver cancer.

For example, AFP is a marker for hepatocellular carcinoma, but shows a high concentration in blood not only in hepatocellular carcinoma but also in chronic hepatitis or hepatic cirrhosis. However, it has been confirmed that AFP (AFP-L3) having a sugar chain (Fucα1-6GlcNac) to which α-L-fucose residue or N-acetylglucosamine residue (bisecting N-acetylglucosamine) is added is frequently observed.

In addition, PSA is known as a prostate cancer marker. Most PSAs observed in serum of a normal person has an N-type sugar chain of which a terminal sialic acid residue α(2,6)-bonds to a second galactose residue from the terminal of the sugar chain. However, it is known that the number of PSAs (hereinafter, described as "α(2,3) sugar chain PSAs") having a sugar chain (Siaα2-3Gal) of which a terminal sialic acid residue α(2,3)-bonds to a second galactose residue from the terminal of the sugar chain increases in serum of a person with prostate cancer (Tajiri M. et al., 2008, Vol. 18, pp. 2-8).

The sugar chain (Siaα2-3Gal) of which a terminal sialic acid residue α(2,3)-bonds to a second galactose residue from the terminal of the sugar chain specifically refers to the following structure.

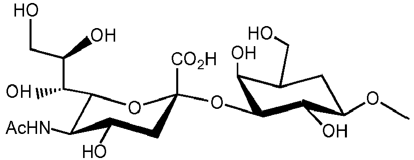

An example of the structure of the sugar chain of the α(2,3) sugar chain PSA is shown in the following formula (I).

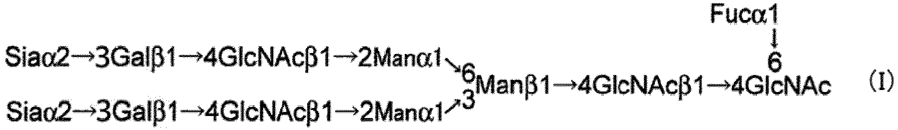

In addition, sugar chains of glycoproteins known as undifferentiation markers or differentiation markers of cells are also exemplified as the substance having a sugar chain according to the embodiment of the present invention.

Examples thereof include (Fucα1-2Galβ1-3GlcNac) and (Fucα1-2Galβ1-3GalNac) as sugar chain structures which have recently become apparent to be present on the surfaces of pluripotent stem cells such as ES cells and iPS cells. In addition, podocalyxin which is glycoconjugate is identified as an undifferentiation sugar chain marker having the sugar chain structure (WO2013/065302).

### Sample Containing Substance Having Sugar Chain

Examples of the sample containing the substance having a sugar chain according to the embodiment of the present invention include living body-derived samples, such as blood, serum, plasma, urine, semen, cerebrospinal fluid, saliva, sweat, ascites, fecal suspension, ascites, a tissue extract, a tissue slice, a tissue biopsy sample, and an organ, of a human or an animal or a sample prepared from these living body-derived samples, and microorganisms such as viruses, bacteria, and cells or a sample prepared from these microorganisms.

Among these, the blood, the serum or the plasma is preferable and the serum is more preferable.

It is possible to use a cell extract, an extract derived from the microorganisms, or a sample prepared from the extracts as the sample containing the substance having a sugar chain according to the embodiment of the present invention.

In addition, some of the bacteria, cells, and the like secrete glycoproteins. In addition, sugar chains are released from glycoproteins present in cell membranes and the like in some cases. For this reason, the living body-derived sample or a culture solution of the microorganisms can be used as the sample containing the substance having a sugar chain according to the embodiment of the present invention.

The sample containing the substance having a sugar chain according to the embodiment of the present invention may be used by being diluted with water or a suitable buffer solution.

Examples of water that can be used for that purpose include purified water such as distilled water and deionized water.

Examples of the buffer solution include a tris buffer solution, a phosphate buffer solution, a Veronal buffer solution, a boric acid buffer solution, a Good's buffer solution, a Tris-HCl buffer solution, an MES buffer solution, an HEPES buffer solution, a boric acid buffer solution, a phosphate buffer solution, a Veronal buffer solution, and a Good's buffer solution. The buffer agent concentrations of these buffer solutions are appropriately selected usually from a range of 5 to 1,000 mM and preferably from a range of 5 to 300 mM. The pH thereof is not particularly limited, but an example thereof includes a range of 5 to 9.

It should be noted that, in the present specification, the "sample containing the substance having a sugar chain" and the "substance having a sugar chain" are used without particular distinction in some cases. That is, in a case where it is simply described as a "sample containing the substance having a sugar chain" in the present specification, the description sometimes includes the meaning of a "substance having a sugar chain".

### Lectin

The lectin used in the present invention may be any lectin which has affinity with a sugar chain of a substance, which has the sugar chain and forms a complex, and recognizes the sugar chain to bond to the sugar chain. A lectin having such properties may be optionally selected from well-known lectins. Among these, a lectin specifically bonding to the sugar chain is preferable.

The origin of a lectin is not limited, and lectins may be derived from animals, plants, fungi, bacteria, viruses, and the like. In addition, lectins may be naturally-derived lectins, recombinant products, or commercially available lectins.

Specific examples of lectins include Lotus tetragonolobus lectin (LTL), Pisum sativum agglutinin (PSA), lens culimaris agglutinin (LCA), Ulex europaeus lectin (UEA-1), Aspergillus oryzae lectin (AOL), Aleuria aurantia lectin (AAL), Agaricus Bisporus (ABA), Jacalin lectin, Peanut agglutinin (PNA), Wisteria floribunda agglutinin (WFA), Amaranthus caudatus agglutinin (ACA), Maclura pomifera lectin (MPA), Helix pomatia agglutinin (HPA), Vicia villosa agglutinin (VVA), Dolichos biflorus agglutinin (DBA), Soybean agglutinin (SBA), Griffonia simplicifolia lectin (GSL-I, GSL-IA4, GSL-II, GSL-IB4), Psophocarpus tetragonolobus lectin (PTL-I), Phaseolus vulgaris agglutinin (PHA-E, PHA-L), Bauhinia purpurea lectin (BPL), Sambucus nigra agglutinin (SNA), Sambucus sieboldiana agglutinin (SSA), Trichosanthes aponica agglutinin (TJA-II, TJA-I), Maackia amurensis lectin (MAA, MAH), Wheat germ lectin (WGA), Datura stramonium agglutinin (DSA), Lycopersicon esculentum lectin (LEL), Solanum tuberosum lectin (STL), Urtica dioica agglutinin (UDA), Pokeweed mitogen lectin (PWM), Erythrina cristagalli agglutinin (ECA), Ricinus communis agglutinin (RCA120), Narcissus pseudonarcissus agglutinin (NPA), Concanavalin A (ConA), Galanthus nivalis agglutinin (GNA), Hippeastrum hybrid lectin (HHL), and Euonymus europaeus lectin (EEL).

In addition, lectins recognizing a sugar chain included in an undifferentiation marker of a pluripotent stem cell are exemplified. An example thereof includes BC2LCN which is a lectin that recognizes (Fucα1-2Galβ1-3GlcNac) or (Fucα1-2Galβ1-3GalNac) which are sugar chains included in podocalyxin known as an undifferentiation marker of a pluripotent stem cell (Tateno H, et al., J. Biol. Chem., Vol. 286, No. 23, pp. 20345-20353, 2011). BC2LCN is a lectin corresponding to an N-terminal domain of BC2L-C protein derived from gram-negative bacteria (*Burkholderia cenocepacia*) (GenBank Accession No. YP_002232818).

A lectin used in the present invention is preferably LCA, MAA, or BC2LCN and more preferably LCA or MAA.

Table 1 below shows an example of lectins used in the present invention, the origin of each lectin (Origin), and an example of a sugar chain structure (Specificity) with which a corresponding lectin has affinity.

**[Table 1]**

| Lectins | Origin | Specificity |
|---|---|---|
| LTL | Lotus tetragonolobus | Fuc *α* 1-3GlcNAc, Sia-Le〈x〉, Le〈x〉 |
| PSA | Pisum sativum | Fuc *α* 1-6GlcNAc, *α*-Man |
| LCA | Lens culinaris | Fuc *α* 1-6GlcNAc, *α*-Man, *α*-Glc |
| UEA-I | Ulex europaeus | Fuc *α* 1-2LacNAcTerminal *α* Fuc, ±Sia-Le〈x〉 |
| AOL | Aspergillus oryzae | Terminal *α* Fuc, ±Sia-Le〈x〉 |
| AAL | Aleuria aurantia | *α* Fuc, ±Sia-Le〈x〉 |
| ABA | Agaricus bisporus | Gal *β* 1-3GalNAc *α*-Thr/Ser (T), sialyl-T |
| Jacalin | Artocarpus integrifolia | Gal *β* 1-3GalNAc *α*-Thr/Ser (T), GalNAc *α* -Thr/Ser (Tn) |
| PNA | Arachis hypogaea | Gal *β* 1-3GalNAc *α*-Thr/Ser (T) |
| WFA | Wisteria floribunda | Terminal GalNAc (e.g., GalNAc *β* 1-4GlcNAc) |
| ACA | Amaranthus caudatus | Gal *β* 1-3GalNAc *α*-Thr/Ser (T) |
| MPA | Maclura pomifera | Gal *β* 1-3GalNAc *α*-Thr/Ser (T), GalNAc *α*-Thr/Ser (Tn) |
| HPA | Helix pomatia Terminal GalNAc | Gal *β* 1-3GalNAc *α*-Thr/Ser (T), GalNAc *α*-Thr/Ser (Tn) |
| VVA | Vicia villosa | *α*-, *β*-linked terminal GalNAc, GalNAc *α*-Thr/Ser (Tn) |
| DBA | Dolichos biflorus | GalNAc *α*-Thr/Ser (Tn), GalNAc *α* 1-3GalNAc |
| SBA | Glycine max | Terminal GalNAc (especially GalNAc *α* 1-3Gal) |
| GSL-1 | Griffonia simplicifolia | *α*-GalNAc, GalNAc *α*-Thr/Ser (Tn), *α*-Gal mixture |
| PTL-I | Psophocarpus tetragonolobus | *α*-GalNAc, Gal |
| GSL-IA4 | Griffonia simplicifolia | *α*-GalNAc, GalNAc *α*-Thr/Ser (Tn) |
| PHA(E) | Phaseolus vulgaris | NA2, bisecting GlcNAc |
| BPL | Bauhinia purpurea alba | Gal *β* 1-3GalNAc, NA3, NA4 |
| SNA | Sambucus nigra | Sia *α* 2-6Gal/GalNAc |
| SSA | Sambucus sieboldiana | Sia *α* 2-6Gal/GalNAc |
| TJA-II | Trichosanthes japonica | Fuc *α* 1-2Gal, *β*-GalNAc 〉 NA3, NA4 |
| MAA | Maackia amurensis | Sia *α* 2-3Gal |
| MAH | Maackia amurensis | Sia *α* 2-3Gal *β* 1-3[Sia *α* 2-6GalNAc] *α*-R |
| TJA-I | Trichosanthes japonica | Sia *α* 2-6Gal *β* 1-4GlcNAc *β*-R |
| WGA | Triticum unlgaris | (GlcNAc)n, multivalent Sia |
| DSA | Datura stramonium | (GlcNAc)n, polyLacNAc. LacNAc (NA3, NA4) |
| LEL | Lycopersicon esculentum | (GlcNAc)n, polyLacNAc |
| STL | Solanum tuberosum | (GlcNAc)n, polyLacNAc |
| UDA | Urtica dioica | (GlcNAc)n, polyLacNAc |
| PWM | Phytolacca americana | (GlcNAc)n, polyLacNAc |
| PHA(L) | Phaseolus vulgaris | Tri- and tetra-antennary complexoligosaccharides |
| ECA | Erythrina cristagalli | Lac/LacNAc |
| RCA120 | Ricinus communis | Lac/LacNAc |
| GSL-II | Griffonia simplicifolia | Agalactosylated N-glycan |
| NPA | Narcissus pseudonarcissus | non-substituted *α* 1-6Man |
| ConA | Canavalia ensiformis | *α*-Man (inhibited by presence of bisecting GlcNAc) |
| GNA | Galanthus nivalis | non-substituted *α* 1-6Man |
| HHL | Hippeastrum hybrid | non-substituted *α* 1-6Man |
| EEL | Euonymus europaeus | Gal *α* 1-3[Fuc *α* 1-2Gal]〉 Gal *α* 1-3Gal |
| GSL-IB4 | Griffonia simplicifolia | *α*-Gal |
| BC2LCN | Burkholderia cenocepacia | Fuc *α* 1-2Gal *β* 1-3GlcNAc, Fuc *α* 1-2Gal *β* 1-3GalNAc |

The lectins may be labeled with a detectable labeling substance.

Examples of the labeling substance used for labeling the lectins include labeling substances such as fluorescent coloring agents (such as fluorescein isothiocyanate (FITC), Cy5, Alexa Flour 647), enzymes (horseradish-derived peroxidase), coenzymes, chemiluminescent substances, radioactive substances (such as 32P, 14C, 1251, 3H, 1311), and biotin. In addition, a labeling substance may directly bond to a lectin or may bond thereto via a suitable spacer.

A lectin may be labeled through a labeling method well known per se depending on the kind of labeling substance.

Furthermore, a lectin may be immobilized on a solid phase such as an insoluble carrier. Any carrier generally used in this field can be used as the insoluble carrier used as a solid phase. Any shape may be used as long as it is generally used in this field, for example, particles (such as latex particles, beads, and magnetic beads), tubes, carbon nanotubes, chips, disk-like pieces, fine particles, thin films, fine tubes, plates, microplates, and filters, and the material thereof is not particularly limited.

In addition, a microchip, a microarray, a macroarray, and a microtiter plate which are produced by immobilizing a lectin on a substrate can also be used in the present invention.

Examples of the method for immobilizing a lectin on an insoluble carrier include immobilization method well known per se depending on the kind of insoluble carrier to be used.

### Method for Forming Complex of Present Invention

The method for forming a complex according to the embodiment of the present invention may be a method through which a "complex, which contains polysaccharides according to the present invention, of a substance having a sugar chain and a lectin" is finally obtained. A method in which a sample containing a substance having a sugar chain is brought into contact with a lectin having affinity with the sugar chain of the substance having a sugar chain in the presence of polysaccharides according to the present invention and by which a "complex solution, which contains polysaccharides according to the present invention, of the substance having a sugar chain and a lectin" is finally obtained is preferable.

Specific examples of the method for forming a complex according to the embodiment of the present invention include:
(i) a method for mixing a sample containing a substance having a sugar chain with polysaccharides according to the present invention or a solution containing the polysaccharides according to the present invention to obtain a mixed solution, and mixing the obtained mixed solution with a lectin having affinity with the sugar chain of the substance having a sugar chain or a solution containing the lectin to cause a reaction therebetween;
(ii) a method for mixing a lectin having affinity with a sugar chain of a substance having a sugar chain or a solution containing the lectin with polysaccharides according to the present invention or a solution containing the polysaccharides according to the present invention to obtain a mixed solution, and mixing the obtained mixed solution with a sample containing the substance having a sugar chain to cause a reaction therebetween;
(iii) a method for mixing a sample containing a substance having a sugar chain with polysaccharides according to the present invention or a solution containing the polysaccharides according to the present invention to obtain a mixed solution, and mixing the obtained mixed solution with a solution containing a lectin having affinity with the sugar chain of the substance having a sugar chain and the polysaccharides to cause a reaction therebetween; and
(iv) a method for mixing a sample containing a substance having a sugar chain with a lectin having affinity with the sugar chain of the substance having a sugar chain or a solution containing the lectin to cause a reaction therebetween, and mixing the obtained reaction solution with polysaccharides according to the present invention or a solution containing the polysaccharides according to the present invention to obtain a mixed solution.

In the method for forming a complex according to the embodiment of the present invention, a sugar chain of a substance having a sugar chain reacts with a lectin in the presence of polysaccharides according to the present invention to form a complex of the substance having a sugar chain and the lectin.

That is, the "reaction" in the method refers to a reaction for forming a complex of a substance having a sugar chain and a lectin.

Among the methods, the methods (i) to (iii) are preferable and the method (i) or (iii) is more preferable.

In a case of performing the method for forming a complex according to the embodiment of the present invention using an automatic analyzer, polysaccharides according to the present invention may be contained in any reagent solution to be used. The solution containing the polysaccharides according to the present invention may be used by being set in one of cells in which a reagent of the automatic analyzer is to be placed. In the measurement using the automatic analyzer, a substance having a sugar chain and a lectin may be brought into contact with each other in the presence of polysaccharides of the present invention to cause a reaction between the sugar chain of the substance having a sugar chain and the lectin.

Examples of a solvent that can be used for the solution containing polysaccharides according to the present invention include water or a buffer solution.

An example of a solvent that can be used for the solution containing a lectin includes a buffer solution.

An example of a solvent that can be used for the solution containing polysaccharides and a lectin according to the present invention includes a buffer solution.

Examples of water that can be used for the solution containing polysaccharides according to the present invention include purified water such as distilled water and deionized water.

Specific examples of the buffer solution that can be used for the solution containing polysaccharides according to the present invention, the solution containing a lectin, and the solution containing a lectin and polysaccharides according to the present invention include a tris buffer solution, a phosphate buffer solution, a Veronal buffer solution, a boric acid buffer solution, a Good's buffer solution, a Tris-HCl buffer solution, an MES buffer solution, an HEPES buffer solution, a boric acid buffer solution, a phosphate buffer solution, a Veronal buffer solution, and a Good's buffer solution. In a case of performing the reaction for forming a complex of a substance having a sugar chain and a lectin using a commercially available kit or a measurement instrument such as an automatic analyzer, a buffer solution attached to the kit, a buffer solution (for example, an HBS-EP buffer for Biacore™ to be described below) exclusive for the automatic analyzer, and the like may be used.

In addition, the buffer agent concentrations of these buffer solutions are appropriately selected usually from a range of 5 to 1,000 mM and preferably from a range of 5 to 300 mM. The pH thereof is not particularly limited, but an example thereof includes a range of 5 to 9.

In addition, reagents which are generally used in this field and inhibit neither the stability of coexisting reagents or the like nor a reaction between a sugar chain and a lectin may be included in these buffer solutions, and examples of the reagents include a buffer agent, a reaction enhancer, proteins, salts, stabilizers such as surfactants, and preservatives. In addition, the concentration thereof may be appropriately selected from the concentration ranges generally used in this field.

Specific examples of a solvent that can be used for the solution containing a substance having a sugar chain and the conditions thereof are as described above.

The concentration of polysaccharides according to the present invention in the solution containing the polysaccharides according to the present invention, the concentration of a lectin in the solution containing the lectin, and the concentration of the substance having a sugar chain in the sample containing the substance having a sugar chain may be any concentration as long as each concentration of a reaction solution when the substance having a sugar chain is brought into contact with the lectin in the presence of polysaccharides according to the present invention to cause a reaction therebetween is within a target concentration range.

For example, the concentration of polysaccharides according to the present invention in the solution containing the polysaccharides according to the present invention is 0.01 (w/v)% to 50 (w/v)%, preferably 0.1 (w/v)% to 25 (w/v)%, more preferably 0.5 (w/v)% to 15 (w/v)%, and still more preferably 0.5 to 10 (w/v)%.

The concentration of a lectin in the solution containing the lectin is 1 µg/mL to 30 mg/mL, preferably 10 µg/mL to 20 mg/mL, and more preferably 1 to 10 mg/mL.

The concentration of a substance having a sugar chain in the sample containing the substance having a sugar chain is 1 pg/mL to 1 mg/mL, preferably 10 pg/mL to 100 µg/mL, and more preferably 1 ng to 50 µg/mL.

In the method for forming a complex according to the embodiment of the present invention, the concentration of polysaccharides according to the present invention in the reaction solution when the substance having a sugar chain is brought into contact with the lectin in the presence of polysaccharides according to the present invention to cause a reaction therebetween varies depending on the kinds of polysaccharides according to the present invention to be used, a method to be performed thereafter, an analysis method, and the like, but is 0.01 (w/v)% to 50 (w/v)%, preferably 0.1 (w/v)% to 25 (w/v)%, more preferably 0.5 (w/v)% to 15 (w/v)%, and still more preferably 0.5 to 10 (w/v)%.

In a case where the substance having a sugar chain is reacted with the lectin in a micro-flow path of capillary electrophoresis apparatus to be described below, the concentration of polysaccharides according to the present invention in the micro-flow path is 0.01 (w/v)% to 50 (w/v)%, preferably 0.1 (w/v)% to 25 (w/v)%, more preferably 1 (w/v)% to 15 (w/v)%, and still more preferably 4 to 10 (w/v)%.

The concentration of the lectin in the reaction solution is 1 µg/mL to 30 mg/mL, preferably 10 µg/mL to 20 mg/mL, and more preferably 1 to 10 mg/mL.

The concentration of the substance having a sugar chain in the reaction solution is 1 pg/mL to 1 mg/mL, preferably 10 pg/mL to 100 µg/mL, and more preferably 1 ng to 50 µg/mL.

It should be noted that, in the case of the method (iii), the concentration of the polysaccharides according to the present invention, the concentration of the lectin, and the concentration of the substance having a sugar chain in the "complex solution, which contains polysaccharides according to the present invention, of the substance having a sugar chain and a lectin" which has been finally obtained may be within the target concentration ranges described above.

The condition that the sample containing a substance having a sugar chain is brought into contact with the lectin to cause a reaction therebetween and the condition that the sample containing a substance having a sugar chain is brought into contact with the lectin in the presence of the polysaccharides according to the present invention to cause a reaction therebetween may be any condition as long as the sugar chain of the substance having a sugar chain and the lectin are sufficiently reacted with each other, thereby forming a complex of the substance having a sugar chain and the lectin.

For example, the temperature when the sample containing the substance having a sugar chain and the lectin is reacted with each other is not particularly limited as long as it is within a range in which the temperature does not suppress the reaction between the sugar chain of the substance having a sugar chain and the lectin, and examples of the range of the temperature thereof include 10°C to 50°C and preferably 20°C to 40°C. In addition, the reaction time varies depending on a lectin to be used and the reaction conditions such as the pH and the temperature. For example, the reaction may be performed for about 1 to 60 minutes and preferably about 1 to 15 minutes.

A method for selecting polysaccharides and lectins according to the present invention which are to be used in the method for forming a complex according to the embodiment of the present invention is as follows.

That is, regarding the polysaccharides according to the present invention to be used in the method for forming a complex according to the embodiment of the present invention, polysaccharides which do not have a sugar chain structure with which a lectin to be used in the method for forming a complex according to the embodiment of the present invention has affinity are selected. That is, in a case of forming a complex of a certain substance having a sugar chain and a lectin, a lectin which has affinity to the sugar chain of the substance having a sugar chain and recognizes and bonds to the sugar chain is selected. Polysaccharides which do not have a sugar chain structure with which the selected lectin has affinity are selected as the polysaccharides according to the present invention

For example, in a case where a complex of a certain substance having a sugar chain and a lectin is formed and the lectin described in Table 1 is selected, a sugar chain according to the present invention which does not have a sugar chain structure (specificity) with which the lectin disclosed in Table 1 has affinity is selected as the polysaccharides according to the present invention.

As a specific example, in a case where AFP (AFP-L3) having a complex of a mutant sugar chain (Fucα1-6GlcNAc) and a lectin is formed, LCA can be used as a lectin having affinity with the mutant sugar chain (Fucαl-6GlcNAc). In this case, polysaccharides having no sugar chain (Fucαl-6GlcNAc, α-Man) with which LCA has affinity are selected as the polysaccharides according to the present invention to allow coexistence during the contact (reaction) between AFP-L3 and LCA. Examples of such polysaccharides according to the present invention include dextran sulfate, chondroitin sulfate, or salts thereof (for example, sodium dextran sulfate and sodium chondroitin sulfate c).

Dextran sulfate has a structure in which a part of dextran in which glucose is polymerized through 1,6-bonding is sulfated. Chondroitin sulfate has a structure in which sulfate bonds to a sugar chain where disaccharides of D-glucuronic acid (GlcUA) and N-acetyl-D-galactosamine (GalNac) are repeated. However, neither dextran sulfate nor chondroitin sulfate has the sugar chain (Fucαl-6GlcNAc, α-Man) with which LCA has affinity.

For example, in a case where a complex of PSA having a mutant sugar chain (Siaα2-3Gal) and a lectin is formed, it is possible to use, for example, MAA as a lectin having affinity with the sugar chain (Siaα2-3Gal). In this case, polysaccharides having no sugar chain (Siaα2-3Galβ1-4GlcNAc) with which MAA has affinity are selected as the polysaccharides according to the present invention to allow coexistence during the reaction between PSA and MAA. Examples of such polysaccharides according to the present invention include dextran sulfate, chondroitin sulfate, or salts thereof (for example, sodium dextran sulfate and sodium chondroitin sulfate c).

In a case where a complex of a pluripotent stem cell having (Fucα1-2Galβ1-3GlcNac) and/or (Fucα1-2Galβ1-3GalNac) as undifferentiation markers on the surfaces of cells is formed, it is possible to use, for example, BC2LCN as a lectin having affinity with the sugar chains. In this case, polysaccharides having neither Fucα1-2Galβ1-3GlcNac) nor (Fucα1-2Galβ1-3GalNac) which are sugar chains with which BC2LCN has affinity are selected as the polysaccharides according to the present invention to allow coexistence during the contact (reaction) between the pluripotent stem cells and BC2LCN. Examples of such polysaccharides according to the present invention include dextran sulfate, chondroitin sulfate, or salts thereof (for example, sodium dextran sulfate and sodium chondroitin sulfate c).

The specific examples of the method for forming a complex according to the embodiment of the present invention are shown below, but are not limited thereto.
(i) A sample such as serum containing 1 pg/mL to 1 mg/mL AFP-L3 is mixed with a buffer solution such as a 5 to 1,000 mM Tris-HCl buffer solution (pH 5 to 9) containing 0.01 (w/v)% to 50 (w/v)% sodium dextran sulfate to obtain a mixed solution. The obtained mixed solution is mixed with 1 µg/mL to 30 mg/mL LCA and a buffer solution such as a 5 to 1,000 mM Tris-HCl buffer solution (pH 5 to 9) containing 0.01 (w/v)% to 50 (w/v)% sodium dextran sulfate to cause a reaction therebetween for 1 to 60 minutes at 10°C to 50°C. The concentration of AFP-L3 in the reaction solution is 1 pg/mL to 1 mg/mL, the concentration of sodium dextran sulfate is 0.01 (w/v)% to 50 (w/v)%, and the concentration of LCA is 1 µg/mL to 30 mg/mL.
(ii) A sample such as serum containing 1 pg/mL to 1 mg/mL AFP-L3 is mixed with a buffer solution such as a 5 to 1,000 mM Tris-HCl buffer solution (pH 5 to 9) containing 0.01 (w/v)% to 50 (w/v)% sodium chondroitin sulfate c to obtain a mixed solution. The obtained mixed solution is mixed with 1 µg/mL to 30 mg/mL LCA and a buffer solution such as a 5 to 1,000 mM Tris-HCl buffer solution (pH 5 to 9) containing 0.01 (w/v)% to 50 (w/v)% sodium chondroitin sulfate c to cause a reaction therebetween for 1 to 60 minutes at 10°C to 50°C. The concentration of AFP-L3 in the reaction solution is 1 pg/mL to 1 mg/mL, the concentration of sodium chondroitin sulfate c is 0.01 (w/v)% to 50 (w/v)%, and the concentration of LCA is 1 µg/mL to 30 mg/mL.
(iii) A sample such as serum containing 1 pg/mL to 1 mg/mL α(2,3) sugar chain free PSA is mixed with a buffer solution such as a 5 to 1,000 mM Tris-HCl buffer solution (pH 5 to 9) containing 0.01 (w/v)% to 50 (w/v)% sodium dextran sulfate to obtain a mixed solution. The obtained mixed solution is mixed with 1 µg/mL to 30 mg/mL MAA and a buffer solution such as a 5 to 1,000 mM Tris-HCl buffer solution (pH 5 to 9) containing 0.01 (w/v)% to 50 (w/v)% sodium dextran sulfate to cause a reaction therebetween for 1 to 60 minutes at 10°C to 50°C. The concentration of α(2,3) sugar chain free PSA in the reaction solution is 1 pg/mL to 1 mg/mL, the concentration of sodium dextran sulfate is 0.01 (w/v)% to 50 (w/v)%, and the concentration of MAA is 1 µg/mL to 30 mg/mL.
(iv) A sample such as serum containing 1 pg/mL to 1 mg/mL α(2,3) sugar chain free PSA is mixed with a buffer solution such as a 5 to 1,000 mM Tris-HCl buffer solution (pH 5 to 9) containing 0.01 (w/v)% to 50 (w/v)% sodium chondroitin sulfate c to obtain a mixed solution. The obtained mixed solution is mixed with 1 µg/mL to 30 mg/mL MAA and a buffer solution such as a 5 to 1,000 mM Tris-HCl buffer solution (pH 5 to 9) containing 0.01 (w/v)% to 50 (w/v)% sodium chondroitin sulfate c to cause a reaction therebetween for 1 to 60 minutes at 10°C to 50°C. The concentration of α(2,3) sugar chain free PSA in the reaction solution is 1 pg/mL to 1 mg/mL, the concentration of sodium chondroitin sulfate c is 0.01 (w/v)% to 50 (w/v)%, and the concentration of MAA is 1 µg/mL to 30 mg/mL.
(v) A sample such as serum containing 1 pg/mL to 1 mg/mL AFP-L3 is mixed with 1 µg/mL to 30 mg/mL LCA and a buffer solution such as a 5 to 1,000 mM Tris-HCl buffer solution (pH 5 to 9) containing 0.01 (w/v)% to 50 (w/v)% sodium dextran sulfate or sodium chondroitin sulfate c to cause a reaction therebetween for 1 to 60 minutes at 10°C to 50°C. The concentration of AFP-L3 in the reaction solution is 1 pg/mL to 1 mg/mL, the concentration of sodium dextran sulfate or sodium chondroitin sulfate c is 0.01 (w/v)% to 50 (w/v)%, and the concentration of LCA is 1 µg/mL to 30 mg/mL.
(vi) A sample such as serum containing 1 pg/mL to 1 mg/mL α(2,3) sugar chain free PSA is mixed with 1 µg/mL to 30 mg/mL MAA and a buffer solution such as a 5 to 1,000 mM Tris-HCl buffer solution (pH 5 to 9) containing 0.01 (w/v)% to 50 (w/v)% sodium dextran sulfate or sodium chondroitin sulfate c to cause a reaction therebetween for 1 to 60 minutes at 10°C to 50°C. The concentration of α(2,3) sugar chain free PSA in the reaction solution is 1 pg/mL to 1 mg/mL, the concentration of sodium dextran sulfate or sodium chondroitin sulfate c is 0.01 (w/v)% to 50 (w/v)%, and the concentration of MAA is 1 µg/mL to 30 mg/mL.
(vii) A sample such as serum containing 1 pg/mL to 1 mg/mL AFP-L3 is mixed with a buffer solution such as a 5 to 1,000 mM HBS-EP buffer solution (pH 5 to 9) containing 0.01 (w/v)% to 50 (w/v)% sodium dextran sulfate or sodium chondroitin sulfate c to obtain a mixed solution. The obtained mixed solution is brought into contact with LCA immobilized on a solid phase such as a sensor chip to cause a reaction therebetween for 1 to 60 minutes at 10°C to 50°C. The concentration of AFP-L3 in the reaction solution is 1 pg/mL to 1 mg/mL, the concentration of sodium dextran sulfate or sodium chondroitin sulfate c is 0.01 (w/v)% to 50 (w/v)%, and the concentration of LCA is 1 µg/mL to 30 mg/mL.
(viii) A sample such as serum containing 1 pg/mL to 1 mg/mL α(2,3) sugar chain free PSA is mixed with a buffer solution such as a 5 to 1,000 mM HBS-EP buffer solution (pH 5 to 9) containing 0.01 (w/v)% to 50 (w/v)% sodium dextran sulfate or sodium chondroitin sulfate c to obtain a mixed solution. The obtained mixed solution is brought into contact with MAA immobilized on a solid phase such as a sensor chip to cause a reaction therebetween for 1 to 60 minutes at 10°C to 50°C. The concentration of α(2,3) sugar chain free PSA in the reaction solution is 1 pg/mL to 1 mg/mL, the concentration of sodium dextran sulfate or sodium chondroitin sulfate c is 0.01 (w/v)% to 50 (w/v)%, and the concentration of MAA is 1 µg/mL to 30 mg/mL.

It should be noted that the reason why the amount of complex of a substance having a sugar chain and a lectin is increased by the method for forming a complex according to the embodiment of the present invention is unclear. However, it is considered that, for example, since a complex which has once been produced is stabilized by causing the substance having a sugar chain to react with the lectin in the presence of polysaccharides according to the present invention, the complex is not separated into the substance having a sugar chain and the lectin again or the amounts thereof separated decreases, which results in increase in the complex even in a case where the substance having a sugar chain and the lectin are reacted with each other in the absence of the polysaccharides according to the present invention.

Alternatively, it is considered that, since affinity of the lectin with the sugar chain of the substance having a sugar chain increases due to the presence of the polysaccharides according to the present invention, the amount of the complex is higher in the case where the substance having a sugar chain is brought into contact with the lectin in the presence of the polysaccharides according to the present invention than in the case where the substance having a sugar chain is brought into contact with the lectin in the absence of the polysaccharides according to the present invention.

### [2] Application of Method for Forming Complex of Present Invention

The method for forming a complex according to the embodiment of the present invention can be used for any measurement method, analysis method, detection method, and the like in which affinity of a lectin with a sugar chain is used.

Examples of the measurement method, the analysis method, or the detection method in which affinity of a lectin with a sugar chain include a surface plasmon resonance method, capillary electrophoresis, lectin affinity chromatography, a lectin microarray method, ELISA, tissue staining, an electrophoresis method, and a flow cytometry.

Among the methods, a surface plasmon resonance method or capillary electrophoresis is preferable
Hereinafter, the method for forming a complex according to the embodiment of the present invention will be described using methods to be applied to the methods as examples.

Specific examples of the components, such as the polysaccharides according to the present invention, the substance having a sugar chain, the sample containing a substance having a sugar chain, and the lectin, according to the present invention which are used for each method, a solvent used in a solution of each component, the concentration of each component in the solution, the reaction conditions, and the like are as described above in the description relating to the method for forming a complex according to the embodiment of the present invention.

In addition, the conditions for selecting the polysaccharides according to the present invention and the lectin to be used in each application method described below are also as described above.

It should be noted that, in a case of carrying out measurement of a substance having a sugar chain, detection of a sugar chain, analysis of a sugar chain structure, and the like after carrying out the method for forming a complex according to the embodiment of the present invention, it is preferable to carry out the measurement and detection in the presence of polysaccharides according to the present invention.

That is, after forming a complex, it is preferable to allow polysaccharides according to the present invention to coexist in a solution containing the complex until target reaction or target measurement and detection are completed (for example, until measurement and detection are completed in a case where measurement and detection of the complex is aimed) The type of the solution and the concentration of polysaccharides in the solution are as described above.

For example, in the case of removing a lectin or a substance having a free sugar chain which have not been used to form a complex in an immunological measurement method to be described below through washing treatment or the like, it is preferable to perform the washing treatment using a washing liquid containing polysaccharides according to the present invention. In addition, it is preferable to measure the amount of the complex in the presence of the polysaccharides according to the present invention.

### Application to Surface Plasmon Resonance Method

The surface plasmon resonance method is an intermolecular interaction analysis system that analyzes (measures) an interaction between biomolecules using an optical phenomenon of so-called surface plasmon resonance (SPR) which occurs in a case where surface plasmon is excited at an interface between metal and liquid.

Examples of methods of applying the method for forming a complex according to the embodiment of the present invention to the surface plasmon resonance method include the following methods.

A lectin having affinity with a sugar chain of a substance having a sugar chain to be measured is immobilized on a sensor chip. A solution containing the sample to be measured and polysaccharides according to the present invention may be prepared to is allowed to flow from a flow path of a surface plasmon resonance spectrometer

Any method through which the solution which is used in the above and contains the sample to be measured and the polysaccharides according to the present invention is finally obtained may be used as the method for obtaining the solution. Examples thereof include a method of adding polysaccharides according to the present invention or a solution with the polysaccharides to the sample or a method of dissolving the sample and the polysaccharides according to the present invention in water or a suitable buffer solution. A "solution containing a sample to be measured and polysaccharides according to the present invention" according to other measurement methods to be described below can also be obtained through the same method.

The measurement performed through the surface plasmon resonance method may be performed under the optimum conditions for the measurement according to the user's manual attached to the surface plasmon resonance spectrometer.

The concentration of polysaccharides according to the present invention in the solution containing the sample, which contains a substance having a sugar chain, and the polysaccharides according to the present invention is 0.01 (w/v)% to 50 (w/v)%, preferably 0.1 (w/v)% to 25 (w/v)%, more preferably 0.5 (w/v)% to 15 (w/v)%, and still more preferably 0.5 to 10 (w/v)%.

If the method for forming a complex according to the embodiment of the present invention is carried out, more complexes of a substance having a sugar chain and a lectin can be obtained as compared with the conventional surface plasmon resonance method. For this reason, it is possible to more accurately obtain the information of the structure of the sugar chain included in the substance with high sensitivity by applying the method for forming a complex according to the embodiment of the present invention to the surface plasmon resonance method in the related art and checking or analyzing a lectin to which the substance having a sugar chain bonds.

In addition, in recent years, a method for detecting and measuring a glycoprotein which has a mutant sugar chain and a marker of a disease through the surface plasmon resonance method has been carried out (Takatoshi Kaya, et al., Anal. Chem., Vol. 87, No. 3, pp. 1797-1803, 2015). In the case where the surface plasmon resonance method using the method for forming a complex according to the embodiment of the present invention is carried out, it is possible to detect the glycoprotein having the mutant sugar chain with high sensitivity. Therefore, the method of the present invention is significantly useful in the field of clinical examination such as determination of a disease or the like.

There is a Biacore™ method as a representative analysis system of the surface plasmon resonance method. In general, the Biacore method is simply called Biacore. In addition, in a case where the "Biacore" is mentioned, in some cases, it refers to a Biacore instrument used for the Biacore analysis system.

An example of a method for detecting AFP-L3 through the Biacore method by the method for forming a complex according to the embodiment of the present invention in which sodium dextran sulfate is used as polysaccharides according to the present invention and LCA is used as a lectin will be described below.

LCA is immobilized on a Biacore-exclusive chip (sensor chip) through a usual method. In addition, a buffer solution (for example, HBS-EP as a Biacore-exclusive running buffer) containing a sample to be measured and sodium dextran sulfate (0.01 (w/v)% to 50 (w/v)%) is prepared. This solution is sent to the sensor chip, for example, at 10°C to 50°C and preferably 20°C to 40°C, at a flow rate of 10 to 50 µL/min to cause a reaction. Accordingly, a complex of AFP-L3 and LCA is formed on the sensor chip in a case where AFP is contained in the sample. Measurement using Biacore is performed 60 to 180 seconds after the start of transferring the solution. The same measurement is performed separately using a buffer, a buffer containing AFP, a buffer containing AFP and polysaccharides according to the present invention, and the like, and background values are adjusted based on the obtained values. The amount of AFP-L3 can be obtained through a usual method based on the obtained measurement results using Biacore.

The amount of AFP-L3 may be determined using a calibration curve obtained by similarly performing the measurement using Biacore in advance using AFP-L3 with a well-known concentration.

### Application to Capillary Electrophoresis

A complex of a substance having a sugar chain and a lectin having affinity with the sugar chain of the substance having a sugar chain is formed through the method for forming a complex according to the embodiment of the present invention, the obtained complex is subjected to separation through capillary electrophoresis. The amount of the substance having a sugar chain can be measured by measuring the amount of the complex.

Examples of the method of applying the method for forming a complex according to the embodiment of the present invention to the capillary electrophoresis include the following [Method 1] and [Method 2].

### [Method 1]

A method 1 is a method for "reacting a sample to be measured with an antibody specifically bonding to a substance having a sugar chain which is to be measured to obtain an antigen-antibody complex of the substance having a sugar chain and the antibody, subjecting the obtained antigen-antibody complex to capillary electrophoresis in the presence of polysaccharides according to the present invention and a lectin having affinity with the sugar chain of the substance having a sugar chain, separating the substance having a sugar chain based on the degree of affinity of the lectin with the sugar chain, and measuring the substance having a sugar chain, to measure the substance having a sugar chain".

One kind of antibody specifically bonding to a substance having a sugar chain may be used as an antibody specifically bonding to a substance having a sugar chain, or two or more kinds of antibodies which are specifically bonding to a substance having a sugar chain, but recognize epitopes different from each other may be used as the antibody specifically bonding to a substance having a sugar chain.

In a case where the substance having a sugar chain is a glycoprotein, an example of the antibody specifically bonding to the substance having a sugar chain includes an antibody specifically bonding to a protein moiety (core protein) of the glycoprotein.

In addition, the antibody may be labeled with a detectable labeling substance.

Antibodies in any cases in which one kind of antibody is used or two or more kinds of antibodies are used may be labeled with a charge carrier molecule such as an anionic substance. An example of the charge carrier molecule includes a nucleic acid chain of DNA or the like.

As the kind of nucleic acid chain to be used for such a purpose, any nucleic acid chain generally used for capillary electrophoresis may be used. In addition, an example of a method for bonding the nucleic acid to an antibody includes a method well known per se.

It should be noted that the antibody may be labeled with both a charged carrier molecule and a detectable labeling substance.

The capillary electrophoresis may be electrophoresis ((micro)chip capillary electrophoresis) performed in a capillary tip.

An electrophoresis solution, electrophoresis conditions, an operation method, a reaction condition, and the like to be used for the capillary electrophoresis according to the present invention may be based on the method well known per se except that the capillary electrophoresis is performed in the presence of polysaccharides according to the present invention.

In a case where capillary electrophoresis is performed using a commercially available fully automated measurement device, polysaccharides according to the present invention and a lectin may be contained in advance in an electrophoresis solution which becomes a mobile phase. An antigen-antibody complex of a substance having a sugar chain and an antibody and a lectin are reacted with each other in a flow path of the fully automated measurement device in the presence of the polysaccharides according to the present invention, and capillary electrophoresis is subsequently carried out. Alternately, capillary electrophoresis may be carried out according to a method described in an instruction manual attached to the device under the conditions described in the instruction manual.

An example of the fully automated measurement device for the capillary electrophoresis includes µTAS WAKO i30 (manufactured by Wako Pure Chemical Industries, Ltd.).

In the case of performing the capillary electrophoresis, the concentration of the polysaccharides according to the present invention in the electrophoresis solution is 0.01 (w/v)% to 50 (w/v)%, preferably 0.1 (w/v)% to 25 (w/v)%, more preferably 1 (w/v)% to 15 (w/v)%, and still more preferably 4 to 10 (w/v)%.

In the case of performing the capillary electrophoresis, it is desirable that the concentration of the lectin in the electrophoresis solution is higher than that of the amount with which a sugar chain of the substance having a sugar chain can completely bond to the lectin while performing separation through the capillary electrophoresis. That is, the concentration of the lectin is 1 µg/mL to 30 mg/mL, preferably 10 µg/mL to 20 mg/mL, and more preferably 1 to 10 mg/mL.

The concentration of polysaccharides according to the present invention in a reaction solution in a case where an antigen-antibody complex is brought into contact with a lectin in the presence of polysaccharides according to the present invention to cause a reaction therebetween and, if necessary, the concentration (concentration in a micro-flow path) of the polysaccharides according to the present invention in the solution in a case where the complex is subjected to separation and detection are 0.01 (w/v)% to 50 (w/v)%, preferably 0.1 (w/v)% to 25 (w/v)%, more preferably 1 (w/v)% to 15 (w/v)%, and still more preferably 4 to 10 (w/v)%.

The concentration of a lectin in a case where an antigen-antibody complex is brought into contact with the lectin in the presence of polysaccharides according to the present invention to cause a reaction therebetween and, if necessary, the concentration of the lectin in the solution in a case where the complex is subjected to separation and detection are 1 µg/mL to 30 mg/mL, preferably 10 µg/mL to 20 mg/mL, and more preferably 1 to 10 mg/mL.

### [Method 2]

A sample to be measured is brought into contact with a labeled lectin obtained by labeling a lectin having a lectin having affinity with a sugar chain of the substance having a sugar chain according to the method for forming a complex according to the embodiment of the present invention in the presence of polysaccharides according to the present invention to cause a reaction therebetween. Subsequently, a complex of [labeled lectin-substance having sugar chain] in the obtained reaction solution was subjected to separation while performing capillary electrophoresis. In a case of performing the capillary electrophoresis, an electrophoresis solution in which polysaccharides according to the present invention are dissolved may be used. It is possible to measure the amount of the substance having a sugar chain in the sample by measuring the labeling substance derived from the complex of [labeled lectin-substance having sugar chain].

The concentration of the polysaccharides according to the present invention and the lectin in the electrophoresis solution, the concentration of the polysaccharides according to the present invention in the reaction solution in the case where a substance having a sugar chain is brought into contact with a labeled lectin in the presence of the polysaccharides according to the present invention to cause a reaction therebetween, if necessary, the concentration (concentration in a micro-flow path) of the polysaccharides according to the present invention in the solution in a case where the complex is subjected to separation and detection, and, the concentration of the lectin, if necessary, the concentration (concentration in a micro-flow path) of the lectin in the solution in a case where the complex is subjected to separation and detection are based on the case of [Method 1].

By carrying out the method for forming a complex according to the embodiment of the present invention and the capillary electrophoresis, it is possible to, for example, subject PSA to separation and measurement according to the difference in its sugar chain.

Among the methods, [Method 1] is preferable.

By applying the method for forming a complex according to the embodiment of the present invention to capillary electrophoresis, it is possible to obtain more complexes of a substance having a sugar chain and a lectin compared to a method in the related art. Therefore, a signal value (such as a peak area) of the peak of the complex detected by the capillary electrophoresis increases. As a result, it is possible to measure the substance having a sugar chain with higher sensitivity.

An example of a method for subjecting α(2,3) sugar chain free PSA to separation and measurement in the [Method 1] will be specifically described below using MAA as a lectin and sodium dextran sulfate or sodium chondroitin sulfate c as polysaccharides according to the present invention.

It should be noted that there is complexed PSA which has bonded to binding protein such as α1-antichymotrypsin or α2-macroglobulin and free PSA which has not bonded to binding protein as PSA such as α(2,3) sugar chain PSA.

First, a sample to be measured and a labeled anti-PSA antibody 1 obtained by labeling an anti-PSA antibody (anti-PSA antibody 1) specifically bonding to free PSA with a detectable labeling substance (for example, a fluorescent substance) were reacted with each other in a liquid phase. In a case where PSA is contained in the sample, PSA bonds to fluorescence-labeled anti-PSA antibody 1 to form the following two types of complexes.
- [Labeled anti-PSA antibody 1 - α(2,3) sugar chain free PSA] complex
- [Labeled anti-PSA antibody 1 - free PSA other than α(2,3) sugar chain free PSA] complex

A reaction solution containing each of the obtained complexes, 2 to 50 µL of a reagent solution containing a DNA-labeled anti-PSA antibody 2 at a concentration of 0.001 to 10 µM, preferably 0.01 to 1 µM obtained by labeling an anti-PSA antibody (anti-PSA antibody 2) specifically bonding to PSA with DNA, an electrophoresis buffer solution, and an internal standard substance (for example, fluorescent substance: HiLyte 647 (manufactured by AnaSpec, Inc.) or the like) are introduced into a capillary having, for example, an inner diameter of 5 to 500 µm, preferably 50 to 200 µm, and more preferably 50 to 100 µm, and a length of 1 to 10 cm through a pressurizing method at 1 to 10 psi for 30 to 60 seconds to cause a reaction therebetween at a warm temperature of 20°C to 40°C for 5 seconds to 30 minutes and preferably 10 seconds to 15 minutes. By this reaction, the following complex 1 and complex 2 are obtained.

Complex 1: Complex of [labeled anti-PSA antibody 1 - α(2,3) sugar chain free PSA - DNA-labeled anti-PSA antibody 2]
Complex 2: Complex of [labeled anti-PSA antibody 1 - free PSA other than α(2,3) sugar chain free PSA - DNA-labeled anti-PSA antibody 2]

Subsequently, a reaction solution containing the complex 1 and the complex 2 is subjected to capillary electrophoresis in an electrophoresis buffer solution (mobile phase) containing MAA (1 µg/mL to 30 mg/mL) and polysaccharides (0.01 w/v)% to 50 (w/v)%) according to the present invention such as sodium dextran sulfate or sodium chondroitin sulfate c. Although the complex 1 interacts with MAA, the complex 2 does not interact with MAA. Therefore, the complex 1 migrates later than the complex 2. For this reason, the appearance position of a peak of the labeling substance derived from the labeled anti-PSA antibody 1 to be detected is different between the complex 1 and the complex 2. Thus, it is possible to recognize a peak of the complex 1 and a peak of the complex 2 based on the position of the peaks.

The amount of α(2,3) sugar chain free PSA can be determined based on the peak area of the complex 1 or the height of the peak.

The number of complexes to be detected is increased by reacting the complex 1 and the complex 2 with MAA in the presence of the polysaccharides according to the present invention in the method, and therefore, it is possible to measure α(2,3) sugar chain free PSA with higher sensitivity.

The anti-PSA antibody 1 (antibody specifically bonding to free PSA) and the anti-PSA antibody 2 (antibody specifically bonding to PSA) used above may be commercially available antibodies.

Examples of commercially available products of the anti-PSA antibody 1 (antibody specifically bonding to free PSA) include Anti-PSA Monoclonal Antibody 8A6 (HyTest Ltd.), Anti-PSA Monoclonal Antibody (PS1) (HyTest Ltd.), Anti-PSA Monoclonal Antibody (CLONE 108) (Anogen), Anti-Prostate Specific AntigenAnti-Prostate Specific Antigen Antibody (PS2) (Abcam plc.), and Anti-Prostate Specific AntigenAnti-Prostate Specific Antigen Antibody (2H9) (Abcam plc.).

The anti-PSA antibody 2 (antibody specifically bonding to PSA) may be an antibody that can bond to both of the free PSA and the complexed PSA. An example thereof includes an antibody specifically bonding to a core protein of PSA. Examples of commercially available products thereof include Anti-PSA Monoclonal Antibody 5A6 (HyTest Ltd.), Anti-PSA Monoclonal Antibody 5G6 (HyTest Ltd.), Anti-PSA Monoclonal Antibody (PS6) (HyTest Ltd.), Anti-Prostate Specific Antigen (EP1588Y) (Abcam plc.), Anti-Prostate Specific Antigen (A67-B/E3) (Abcam plc.), Anti-Prostate Specific Antigen (35H9) (Abcam plc.), Anti-Prostate Specific Antigen (KLK3/801) (Abcam plc.), Anti-Prostate Specific Antigen (3E6) (Abcam plc.), Anti-Prostate Specific Antigen (8301) (Abcam plc.), Anti-Prostate Specific Antigen (A5D5) (Abcam plc.), Anti-Prostate Specific Antigen (PSA 28/A4) (Abcam plc.), and Anti-Prostate Specific Antigen (1H12) (Abcam plc.).

### Application to Lectin Affinity Chromatography

Lectin affinity chromatography is often used as a method for purifying glycoproteins, glycopeptides, sugar chains, and the like.

In the lectin affinity chromatography, a sample is allowed to flow through a column filled with filler in which a lectin having affinity to a sugar chain of a substance having a sugar chain to be measured is immobilized on a solid phase such as agarose. In a case where there is a substance having a sugar chain to be measured in the sample, the target substance having a sugar chain is separated from the sample based on the delay in elution of the substance having a sugar chain, which occurs due to the interaction between the sugar chain and the lectin, and is measured.

An example of the method of applying the method for forming a complex according to the embodiment of the present invention to lectin affinity chromatography includes a usual method in which lectin affinity chromatography is performed using a mobile phase containing polysaccharides according to the present invention.

The concentration of the polysaccharides according to the present invention in the mobile phase is 0.01 (w/v)% to 50 (w/v)%, preferably 0.1 (w/v)% to 25 (w/v)%, more preferably 0.5 (w/v)% to 15 (w/v)%, and still more preferably 0.5 to 10 (w/v)%.

It is possible to more efficiently separate the substance having a sugar chain than in the related art by applying the method for forming a complex according to the embodiment of the present invention to the lectin affinity chromatography.

### Application to Lectin Microarray Method

It is possible to apply the method for forming a complex according to the embodiment of the present invention to a lectin microarray method developed by Research Center for Medical Glycoscience, National Institute of Advanced Industrial Science and Technology.

The lectin array (lectin microarray) is an array obtained by arranging several ten kinds of lectins having different specificities (affinities) on slide glass in a spot shape for immobilization.

Examples of the method of applying the method for forming a complex according to the embodiment of the present invention to the lectin microarray method include the following [Method 1] and [Method 2].

### [Method 1]

A solution containing polysaccharides according to the present invention and a substance having a sugar chain is reacted with a lectin microarray. Then, the microarray is reacted with a fluorescence-labeled antibody obtained by fluorescently labeling an antibody specifically bonding to the substance having a sugar chain. Subsequently, it is possible to recognize a lectin, to which the substance having a sugar chain bonds, by detecting the fluorescence derived from the fluorescence-labeled antibody through the same method as described above, and therefore, it is possible to analyze the sugar chain of the substance having a sugar chain based on the results.

### [Method 2]

A solution containing polysaccharides according to the present invention and a labeled substance of a substance having a sugar chain which is obtained by labeling the substance having a sugar chain with a detectable labeling substance such as a fluorescent substance is allowed to react with a lectin microarray. Then, excitation light is radiated to generate an evanescent field. Subsequently, it is possible to recognize a lectin, to which the substance having a sugar chain bond, by detecting a signal derived from the labeled substance of the substance having a sugar chain, and therefore, it is possible to analyze the sugar chain of the substance having a sugar chain based on the results.

The concentration of the polysaccharides according to the present invention in the solution containing the polysaccharides according to the present invention and the substance having a sugar chain is 0.01 (w/v)% to 50 (w/v)%, preferably 0.1 (w/v)% to 25 (w/v)%, more preferably 0.5 (w/v)% to 15 (w/v)%, and still more preferably 0.5 to 10 (w/v)%.

The measurement using a lectin microarray may be carried out according to the protocols disclosed, for example, in MICROARRAY METHODS AND PROTOCOLS (CRCPRESS), edited by Robert S. Matson, "Chapter 9: Lectin Microarrays", Masao Yamada, p. 141,2009.

Among the methods, [Method 1] is preferable.

The lectin microarray method is a method capable of detecting fluorescence of a lectin array without performing a washing operation. Therefore, even in a case where a sugar chain has weak affinity with a lectin, a larger amount of sugar chain-lectin complex is maintained on the microarray by applying the method for forming a complex according to the embodiment of the present invention to the lectin microarray method. For this reason, the information on the sugar chain can be obtained with higher accuracy.

### Application to Immunological Measurement Method (Sandwich Method)

In a case of applying the method for forming a complex according to the embodiment of the present invention to an immunological measurement method (sandwich method), measurement may be performed based on a well-known immunological measurement method (sandwich method) except that a substance having a sugar chain is reacted with a lectin in the presence of polysaccharides according to the embodiment of the present invention.

Examples of the method of applying the method for forming a complex according to the embodiment of the present invention to the sandwich method using a solid phase include the following [Method 1] and [Method 2].

### [Method 1]

A lectin having affinity with a sugar chain of a substance having a sugar chain to be measured is immobilized on a solid phase. A solution containing the sample to be measured and polysaccharides according to the present invention is brought into contact with the solid phase to cause a reaction therebetween. Alternatively, solid phase may be brought into contact with the solution containing the polysaccharides according to the present invention and the sample to be measured in this order to cause a reaction therebetween, or the solid phase may be brought into contact with the sample to be measured and the solution containing the polysaccharides according to the present invention in this order to cause a reaction therebetween.

After the solid phase is subjected to washing treatment, a solution of a labeled antibody obtained by labeling an antibody specifically bonding to the substance having a sugar chain to be measured with a detectable labeling substance is brought into contact with the solid phase to cause a reaction therebetween. In a case where the substance having a sugar chain is a glycoprotein, an antibody specific to a core protein thereof may be used. The solid phase is subjected to washing treatment to remove an unreacted labeled antibody.

The amount of the labeling substance derived from the complex of [lectin-substance having sugar chain-labeled antibody] formed on the solid phase is measured through the measurement method corresponding to the labeling substance of the labeled antibody. The substance having a sugar chain is measured based on the results.

### [Method 2]

An antibody specifically bonding to a substance having a sugar chain to be measured is immobilized on a solid phase. In a case where the substance having a sugar chain is a glycoprotein, an antibody specific to a core protein thereof may be used.

Thereafter, the sample to be measured is brought into contact with the solid phase to cause a reaction therebetween.

Subsequently, after the solid phase is subjected to washing treatment, a solution containing polysaccharides according to the present invention and a labeled lectin obtained by labeling a lectin having affinity with a sugar chain of a substance having a sugar chain to be measured with a detectable labeling substance is brought into contact with the solid phase to cause a reaction therebetween. Alternatively, a the solid phase may be brought into contact with a solution containing the polysaccharides according to the present invention and the labeled lectin obtained by labeling a lectin having affinity with a sugar chain of a substance having a sugar chain with a detectable labeling substance in this order to cause a reaction therebetween, or the solid phase is brought into contact with a solution containing the labeled lectin and the solution containing the polysaccharides according to the present invention in this order to cause a reaction therebetween.

The solid phase is subjected to washing treatment to remove an unreacted labeled lectin or the like.

The amount of the labeling substance derived from the complex of [lectin-substance having sugar chain-labeled lectin] formed on the solid phase is measured through the method corresponding to the labeling substance of the labeled lectin. The substance having a sugar chain is measured based on the results.

In [Method 1] and [Method 2], the concentration of the polysaccharides according to the present invention in the reaction solution in the case where the sample to be measured is reacted with the lectin is 0.01 (w/v)% to 50 (w/v)%, preferably 0.1 (w/v)% to 25 (w/v)%, more preferably 0.5 (w/v)% to 15 (w/v)%, and still more preferably 0.5 to 10 (w/v)%.

The washing liquid which has been used in [Method 1] and [Method 2] in the case of subjecting the solid phase after each reaction and the solution of the labeled antibody which has been used in [Method 1] preferably contain polysaccharides according to the present invention. The concentration of the polysaccharides according to the present invention in the washing liquid is as described above.

In addition, it is preferable to measure the amount of the labeling substance derived from the complex of [lectin-substance having sugar chain-labeled lectin] in the presence of the polysaccharides according to the present invention. The concentration of the polysaccharides according to the present invention during the measurement may be the same as that in the reaction solution.

Among the methods, [Method 2] is more preferable.

It is possible to obtain more complexes of the substance having a sugar chain and the lectin in the case where the method for forming a complex according to the embodiment of the present invention is applied to the immunological measurement method, and therefore, it is possible to measure the substance having a sugar chain with higher sensitivity.

### Application to Electrophoresis Method

After subjecting a sample to be measured to gel electrophoresis through a usual method, the sample is transferred onto a membrane such as a PVDF membrane. Subsequently, after appropriately blocking the membrane, the obtained membrane is immersed in a solution containing polysaccharides according to the present invention and a labeled lectin obtained by labeling a lectin having affinity with a sugar chain of a substance having a sugar chain with a detectable labeling substance to form a complex of the substance having a sugar chain and the labeled lectin on the membrane. Subsequently, it is possible to detect and measure the substance having a sugar chain with high sensitivity by detecting the labeling substance through the measurement method corresponding to the labeling substance.

In a case of using a biotin-labeled lectin obtained by labeling a lectin having affinity with a sugar chain of a substance having a sugar chain with biotin as a labeled lectin, the following procedure is performed. First, a sample containing a substance having a sugar chain is subjected to gel electrophoresis, and is then transferred onto a membrane. The membrane is immersed in a solution containing a biotin-labeled lectin and polysaccharides according to the present invention to form a complex of the substance having a sugar chain and the biotin-labeled lectin on the membrane. Subsequently, the membrane is immersed in an HRP-labeled avidin solution (which may contain the polysaccharides according to the present invention). Furthermore, the membrane is immersed in a color developing solution to develop color. The substance having a sugar chain is detected and measured by detecting the color development.

The concentration of the polysaccharides according to the present invention in the solution containing the labeled lectin and the polysaccharides according to the present invention used above or the solution containing the biotin-labeled lectin and the polysaccharides according to the present invention used above is 0.01 (w/v)% to 50 (w/v)%, preferably 0.1 (w/v)% to 25 (w/v)%, more preferably 0.5 (w/v)% to 15 (w/v)%, and still more preferably 0.5 to 10 (w/v)%.

The amount of the complex of the substance having a sugar chain and the labeled lectin which is formed on the membrane is increased by applying the method for forming a complex according to the embodiment of the present invention to the electrophoresis method. Therefore, a signal value derived from the labeled lectin of the complex increases. As a result, it is possible to detect or measure the substance having a sugar chain with higher sensitivity.

### Application to Tissue Staining

A tissue slice prepared through a usual method is immersed in a solution containing polysaccharides according to the present invention and a labeled lectin obtained by labeling a lectin, which has affinity with a sugar chain to be detected, with a detectable labeling substance such as a fluorescent substance or a radioactive substance to form a complex of the sugar chain and the labeled lectin on the tissue slice. Accordingly, it is possible to efficiently label the tissue slice.

The concentration of the polysaccharides according to the present invention in the solution containing the polysaccharides according to the present invention and the labeled lectin used in the tissue staining is 0.01 (w/v)% to 50 (w/v)%, preferably 0.1 (w/v)% to 25 (w/v)%, more preferably 0.5 (w/v)% to 15 (w/v)%, and still more preferably 0.5 to 10 (w/v)%.

### Application to Flow Cytometry

Usual flow cytometry in which a lectin obtained by labeling a lectin, having affinity with a (target) sugar chain which is present in a cell membrane and to be examined, with a detectable labeling substance is bonded to a cell and a cell separation device (cell sorter) is used is carried out, it is possible to fractionate subpopulations of cells based on the kinds of sugar chains on the surfaces of the cells (on the cell membrane) using specificity of a lectin to a sugar chain.

Examples of methods of applying the method for forming a complex according to the embodiment of the present invention to the method include the following methods.

A cell is brought into contact with a solution containing polysaccharides according to the present invention and a labeled lectin obtained by labeling a lectin having affinity with a target sugar chain (which is considered to be possessed by the cell on a cell membrane) with a fluorescent substance to form a complex of the sugar chain on the cell membrane and the fluorescence-labeled lectin. Thereafter, a cell to which the labeled lectin has bonded and a cell to which the labeled lectin has not bonded are separated from each other through a method for detecting fluorescence in which a usual cell sorter is used. A separation liquid in the case of separating cells from each other with the cell sorter may contain polysaccharides according to the present invention.

The concentration of polysaccharides according to the present invention in the case of bringing cells into contact with the solution containing the fluorescence-labeled lectin and the polysaccharides according to the present invention is 0.01 (w/v)% to 50 (w/v)%, preferably 0.1 (w/v)% to 25 (w/v)%, more preferably 0.5 (w/v)% to 15 (w/v)%, and still more preferably 0.5 to 10 (w/v)%.

In addition, the concentration of polysaccharides according to the present invention in a separation liquid in which the polysaccharides according to the present invention dissolve in the case of separating cells from each other with the cell sorter using the separation liquid is 0.01 (w/v)% to 50 (w/v)%, preferably 0.1 (w/v)% to 25 (w/v)%, more preferably 0.5 (w/v)% to 15 (w/v)%, and still more preferably 0.5 to 10 (w/v)%.

The amount of complex, which can be obtained, of a lectin and a cell having a substance having a sugar chain is increased by applying the method for forming a complex according to the embodiment of the present invention to flow cytometry. Therefore, the amount of cells to which the lectin is bonded and which can be obtained through separation increases. That is, cells having target sugar chains can be efficiently collected.

In addition, in regenerative medicine, quality control of cells to be used is essential. In recent years, it has become clear that it is possible to identify differentiation or undifferentiation of ES cells and iPS cells by analyzing sugar chains on the surfaces of cells. In addition, a lectin specifically recognizing a sugar chain of an undifferentiated cell was found (Tateno H, et al., J. Biol. Chem., Vol. 286, No. 23, pp. 20345-20353, 2011). A technique for managing the quality of undifferentiated cells or differentiated cells has been developed based on the findings (WO2013/065302A and WO2013/128914A).

For example, a complex of an undifferentiated cell and a labeled lectin is formed through the method for forming a complex according to the embodiment of the present invention using a labeled lectin obtained by labeling a lectin specifically recognizing a sugar chain of an undifferentiated cell with a detectable labeling substance. Subsequently, it is possible to efficiently classify undifferentiated cells and differentiated cells by separating the undifferentiated cells and the differentiated cells from each other with a cell sorter.

An example of other techniques to which the reaction for forming a complex of the present invention can be applied includes a method for determining blood types (subtypes) using a lectin. The determination may be carried out using one obtained by adding the polysaccharides according to the present invention to any of blood type determination reagents.

Among the methods exemplified above, it is preferable to apply the method for forming a complex according to the embodiment of the present invention to a plasmon resonance method and capillary electrophoresis and it is particularly preferable to apply the method thereof to the plasmon resonance method.

### [3] Method for Measuring Substance Having Sugar Chain of Present Invention

The method for measuring a substance having a sugar chain of the present invention is a "method for measuring a substance having a sugar chain, including: forming the complex of the substance having a sugar chain and the lectin in the method for forming a complex according to the embodiment of the present invention to measure an amount of the complex".

The details of the method for forming a complex according to the embodiment of the present invention in the method for measuring a substance having a sugar chain of the present invention are as described in the "method for forming a complex according to the embodiment of the present invention" above.

After forming a complex of a substance having a sugar chain and a lectin through the method for forming a complex according to the embodiment of the present invention, the substance having a sugar chain may be measured by measuring the amount of the complex. Specific examples of the method for measuring the complex include a surface plasmon resonance method, capillary electrophoresis, lectin affinity chromatography, a lectin microarray method, an immunological measurement method such as a sandwich method, and an electrophoresis method. The specific conditions, methods, or the like are as described above in the description of each method.

### [4] Enhancer for Forming Complex of Present Invention

The enhancer for forming a complex of the present invention is an "enhancer for forming a complex of a substance having a sugar chain and a lectin having affinity with the sugar chain of the substance having a sugar chain, the enhancer containing polysaccharides of the present invention".

An example of the polysaccharides according to the present invention contained in the enhancer for forming a complex of the present invention includes a water-soluble polysaccharide having no N-acetylglucosamine or a water-soluble compound having a polysaccharide having no N-acetylglucosamine which has been described above in the description relating to the method for forming a complex according to the embodiment of the present invention. Preferred aspects and specific examples thereof are also as described above.

Specific examples thereof include dextran sulfate or a salt thereof, and sodium chondroitin sulfate c or a salt thereof.

In a case where the enhancer for forming a complex of the present invention is a solution, the concentration of polysaccharides according to the present invention in the solution of the enhancer for forming a complex according to the present invention is 0.01 (w/v)% to 50 (w/v)%, preferably 0.1 (w/v)% to 25 (w/v)%, more preferably 0.5 (w/v)% to 15 (w/v)%, and still more preferably 0.5 to 10 (w/v)%.

In the case where the enhancer for forming a complex of the present invention is a solution, specific examples of a solvent that can be used for the solution include water or a buffer solution.

Water that can be used as a solvent is not particularly limited as long as it is used in this field, and specific examples thereof include purified water such as distilled water and deionized water.

Examples of the buffer solution that can be used as a solvent include a tris buffer solution, a phosphate buffer solution, a Veronal buffer solution, a boric acid buffer solution, a Good's buffer solution, a Tris-HCl buffer solution, an MES buffer solution, an HEPES buffer solution, a boric acid buffer solution, a phosphate buffer solution, a Veronal buffer solution, and a Good's buffer solution.

In addition, the buffer agent concentrations of these buffer solutions are appropriately selected usually from a range of 5 to 1,000 mM and preferably from a range of 5 to 300 mM. The pH thereof is not particularly limited, but an example thereof includes a range of 5 to 9.

The enhancer for forming a complex of the present invention may contain a lectin. Specific examples of the lectin are as described above in the description of the method for forming a complex according to the embodiment of the present invention. Examples thereof include LCA, MAA, and BC2LCN. In addition, the concentration of a lectin in the enhancer for forming a complex according to the present invention is 1 µg/mL to 30 mg/mL, preferably 10 µg/mL to 20 mg/mL, and more preferably 1 to 10 mg/mL.

In a case where the enhancer for forming a complex of the present invention contains polysaccharides of the present invention and a lectin, polysaccharides of the present invention are selected from those having no sugar chain with which the lectin has affinity as described above in the method for forming a complex according to the embodiment of the present invention.

Specific examples of the enhancer for forming a complex of the present invention which contains polysaccharides of the present invention and a lectin include enhancer having the following compositions.
(1) An enhancer for forming a complex obtained by dissolving 0.01 (w/v)% to 50 (w/v)% dextran sulfate or a salt thereof and 1 µg/mL to 30 mg/mL LCA in a buffer solution such as a 5 to 1,000 mM Tris-HCl buffer solution (pH 5 to 9)
(2) An enhancer for forming a complex obtained by dissolving 0.01 (w/v)% to 50 (w/v)% dextran sulfate or a salt thereof and 1 µg/mL to 30 mg/mL MAA in a buffer solution such as a 5 to 1,000 mM Tris-HCl buffer solution (pH 5 to 9)
(3) An enhancer for forming a complex obtained by dissolving 0.01 (w/v)% to 50 (w/v)% chondroitin sulfate c or a salt thereof and 1 µg/mL to 30 mg/mL MAA in a buffer solution such as a 5 to 1,000 mM Tris-HCl buffer solution (pH 5 to 9)

Reagents which are generally used in this field and inhibit neither the stability of coexisting reagents or the like nor a reaction between a sugar chain and a lectin may be included in the enhancer for forming a complex of the present invention, and examples of the reagents include a buffer agent, a reaction enhancer, proteins, salts, stabilizers such as surfactants, and preservatives. In addition, the concentration thereof may be appropriately selected from the concentration ranges generally used in this field.

Furthermore, in the user's manual of the enhancer for forming a complex may be attached with the enhancer for forming a complex of the present invention. The "manual" means an instruction manual, attached document, a pamphlet (leaflet), and the like of the enhancer for forming a complex of the present invention in which the characteristics of the enhancer for forming a complex of the present invention, the method for using the enhancer, and the like are substantially disclosed in sentences or using diagrams.

Hereinafter, the present invention will be described in more detail using examples, but is not limited to these examples.

### Examples

### Example 1: Detection of AFP-L3

### (1) Measurement Instrument and the like

Measurement instrument: Biacore X (manufactured by GE Healthcare UK Ltd.)
Chip: Sensor Chip CM5 (manufactured by GE Healthcare UK Ltd.)
Running buffer: a HBS-EP buffer (10 mM HEPES, 0.15 M NaCl, 3 mM EDTA, 0.005% Surfactant P 20, pH 7.4, manufactured by GE Healthcare UK Ltd.)

### (2) Reagent and the like

AFP-L3 (LCA bonding fraction of α-Fetoprotein manufactured by Wako Pure Chemical Industries, Ltd.) was used as a substance having a sugar chain.

Sodium dextran sulfate (M.W. 36,000 to 50,000, manufactured by Wako Pure Chemical Industries, Ltd.) and Sodium chondroitin sulfate c (M.W. 40,000 to 80,000, manufactured by Wako Pure Chemical Industries, Ltd.) were used as polysaccharides according to the present invention.

### (3) Sample Solution

The following sample solutions were prepared using an HBS-EP buffer and the reagents.
- 28 µg/mL AFP-L3 Solution containing 1 µM (4 w/v%) sodium dextran sulfate
- 28 µg/mL AFP-L3 Solution containing 1 µM (1 w/v%) sodium chondroitin sulfate c
- 28 µg/mL AFP-L3 Solution

### (4) Immobilization of LCA on Sensor Chip

Lens culinaris agglutinin (LCA) was immobilized on a sensor chip which is Sensor Chip CM5 (CM sensor chip manufactured by GE Healthcare UK Ltd.) using an amine coupling kit (manufactured by GE Healthcare UK Ltd.).

### (5) Measurement using Biacore

The following measurement was performed using Biacore X (manufactured by GE Healthcare UK Ltd.).

60 µL of each of the sample solutions prepared in (3) was running slowly under the conditions of a temperature of 25°C, a flow rate of 30 µL, and an association time of 120 seconds and allowed to flow on the sensor chip on which LCA was immobilized to cause a reaction between AFP-L3 and LCA. Immediately after transferring the solution, continuous measurement using Biacore X was performed. Subsequently only the HBS-EP buffer was running for 180 seconds (dissociation time). The obtained measurement results were analyzed using BIAevaluation (Version 4.1) which is Biacore-exclusive analysis software to obtain sensorgrams.

### (6) Results

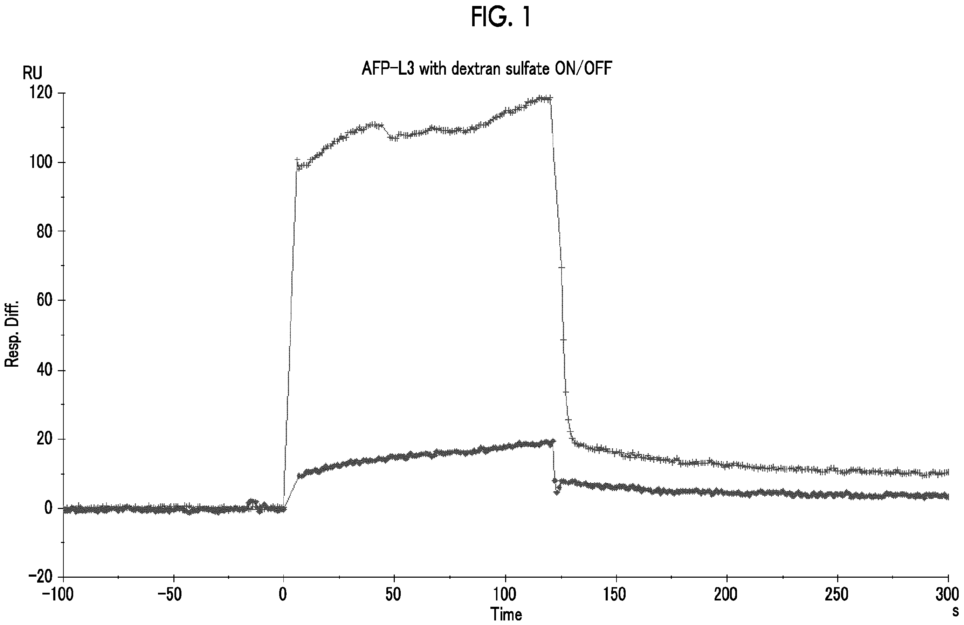

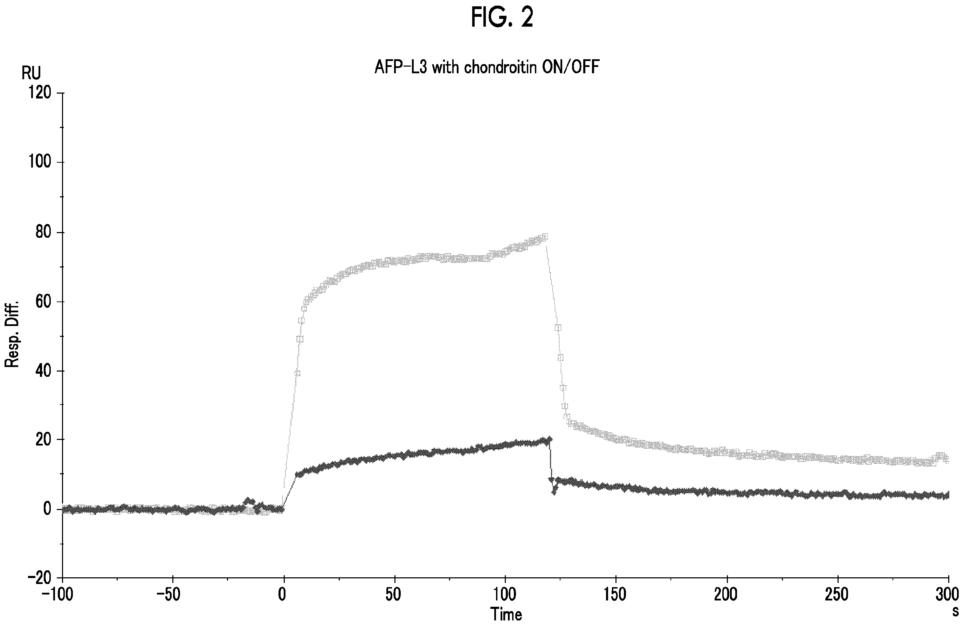

The obtained sensorgrams are shown in Figs. 1 and 2.

In Figs. 1 and 2, the lateral axis represents time (s (seconds)) and the vertical axis represents a response value (resonance unit, RU). The magnitude of RU reflects the amount of complex of AFP-L3 and LCA.

In addition, in Fig. 1, + represents the results obtained using an AFP-L3 solution containing sodium dextran sulfate and ◆ represents the results obtained using an AFP-L3 solution containing no sodium dextran sulfate.

In Fig. 2, □ represents the results obtained using an AFP-L3 solution containing sodium chondroitin sulfate c and ◆ represents the results obtained using an AFP-L3 solution containing no sodium chondroitin sulfate c.

It should be noted that the reaction between AFP-L3 and LCA was performed for 120 seconds, and then, only the HBS-EP buffer was allowed to flow on the sensor chip on which LCA was immobilized. Accordingly, in Figs. 1 and 2, only the HBS-EP buffer was running to the sensor chip from a point in time of 120 sec on the lateral axis (which is called "dissociation" in Biacore).

As is as is apparent from the Figs. 1 and 2, in a case where AFP-L3 was reacted with LCA in the presence of sodium dextran sulfate or sodium chondroitin sulfate c, the amount of complex of AFP-L3 and LCA during dissociation was larger than in a case where AFP-L3 was reacted with LCA in a state where there is no sodium dextran sulfate nor sodium chondroitin sulfate c. That is, in a case where AFP-L3 is brought into contact with LCA in the presence of polysaccharides of the present invention to cause a reaction therebetween, it can be seen that the amount of complex of AFP-L3 and LCA increases compared to a case where AFP-L3 is brought into contact with LCA in the absence of polysaccharides of the present invention to cause a reaction therebetween.

### Example 2. Detection 1 of α(2,3) sugar chain free PSA

### (1) Preparation of Sample and Reagent Solutions

### 1) Preparation of DNA-Labeled Anti-PSA Antibody

A PSA antibody Fab' fragment to which DNA was conjugated was prepared according to the following procedure.

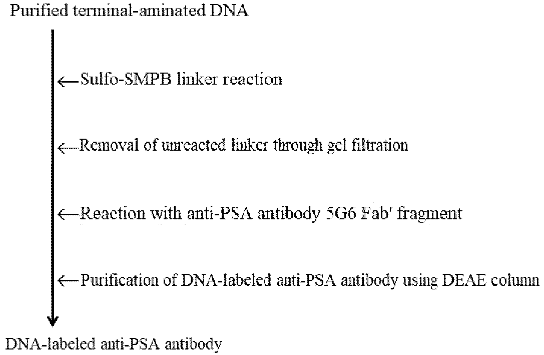

That is, first, a 250-bp DNA fragment into which an NH₂ group is introduced at the 5' terminal is purified through a usual method (purified terminal-aminated DNA), and then, the NH₂ group introduced into the DNA fragment was reacted with a succinimide group of a sulfosuccinimidyl 4-(p-maleimidophenyl)butyrate (Sulfo-SMPB) linker (linker which had a succinimide group and a maleimide group and was manufactured by Pierce) through a usual method. Subsequently, gel filtration was performed to remove an unreacted linker to obtain a 250-bp DNA fragment to which the linker was conjugated. The obtained linker-conjugated 250-bp DNA fragment was reacted with an anti-PSA antibody 5G6 Fab' fragment which had been previously prepared according to a usual method using an anti-human PSA mouse monoclonal antibody (Anti-PSA Monoclonal Antibody 5G6, manufactured by HyTest Ltd.). The resulting reactant was purified using a DEAE column to prepare an anti-PSA antibody 5G6 Fab' fragment to which the 250-bp DNA fragment was conjugated (hereinafter, referred to as a "DNA-labeled anti-PSA antibody").

It should be noted that the Anti-PSA Monoclonal Antibody 5G6 used is an antibody having affinity with human PSA and can be bonded to both complexed PSA and free PSA. That is, the antibody can be bond to both "α(2,3) sugar chain free PSA" and "free PSA other than α(2,3) sugar chain free PSA".

### 2) Preparation of Fluorescence-Labeled Anti-Free PSA Antibody

An epitope of PSA different from that of the Anti-PSA Monoclonal Antibody 5G6 was recognized and an anti-human PSA monoclonal antibody (Anti-PSA Monoclonal Antibody 8A6 manufactured by HyTest Ltd.) specifically bonding to only free PSA was treated through a usual method to obtain an anti-PSA antibody 8A6 Fab' fragment. A fluorescent substance HiLyte 647 (manufactured by AnaSpec, Inc.) was introduced into an amino group of the obtained fragment to obtain a HiLyte 647-labeled anti-free PSA antibody 8A6 Fab' fragment (hereinafter, referred to as a "fluorescence-labeled anti-free PSA antibody").

### (2) Electrophoresis (Microchip Capillary Electrophoresis)

Microchip capillary electrophoresis was performed according to the procedure shown below using a fully automated fluorescence immunoassay device µTAS WAKO i30 (manufactured by Wako Pure Chemical Industries, Ltd.) according to the instruction manual.

### 1) Preparation of Electrophoresis Sample A

Recombinant free PSA [hereinafter, abbreviated as "r-free PSA" which contains recombinant α(2,3) free PSA (hereinafter, abbreviated as rα(2,3) free PSA) and free PSA other than the recombinant α(2,3) free PSA] was acquired according to a method disclosed in "2. Materials and methods (2.7 Forced expression of FLAG-tag-fused S2, 3PSA)" of Yoneyama T. et al., Biochem. Biophys. Res. Commun., Vol. 448, No. 4, pp. 390-396, 2014. The concentration of PSA in the acquired r-free PSA solution was measured and a sample solution of which the concentration became a PSA protein concentration of 1 ng/mL was obtained by diluting the r-free PSA solution with PBS(-) (manufactured by Wako Pure Chemical Industries, Ltd.). 2 µL of the obtained sample solution, 1 µL of the 1 µM fluorescence-labeled anti-free PSA antibody prepared in 2) of (1) above, and 7 µL of an electrophoresis buffer solution 1 [which contains 5% (w/v) polyethylene glycol (PEG20000), 3%(w/v) glycerol, 150 mM NaCl, 0.01% BSA, 75 mM Tris-HCl, and 10 mM MES and has a pH of 7.5] were added to a 0.5 mL tube and mixed with each other to prepare 10 µL of a reaction solution.

It should be noted that the final concentration of the fluorescence-labeled anti-free PSA antibody in the reaction solution is 100 nM.

The solution which was obtained through the reaction and contains a [fluorescence-labeled anti-free PSA antibody - r-free PSA] complex (that is, a solution containing a [fluorescence-labeled anti-free PSA antibody - rα(2,3) sugar chain free PSA] complex and a solution containing a [fluorescence-labeled anti-free PSA antibody - free PSA other than rα(2,3) sugar chain free PSA] compound) (10 µL) was regarded as an electrophoresis sample A.

### 2) Preparation of Electrophoresis Reagent Solutions

The following reagent solutions were prepared.

### • Electrophoresis buffer solution 2 (containing MAA and sodium chondroitin sulfate c)

A 75 mM Tris-HCl buffer (pH 7.5) containing 5.0% (w/v) polyethylene glycol (PEG8000), 3% (w/v) glycerol, 10 mM NaCl, and 0.01% BSA was prepared. MAA (manufactured by VECTOR) and sodium chondroitin sulfate c (manufactured by Wako Pure Chemical Industries, Ltd.), which was as polysaccharides according to the present invention, were added to and mixed with the 75 mM Tris-HCl buffer so that the final concentration (concentration in an electrophoresis buffer solution 2) of MAA became 4 mg/mL and each final concentration (w/v) (concentration in an electrophoresis buffer solution 2) of sodium chondroitin sulfate c became 0%, 2.8%, 3.4%, 3.5%, 3.6%, 3.8%, 4.1%, 4.3%, and 4.5% to prepare each electrophoresis buffer solution 2.

### • Electrophoresis buffer Solution 3

A buffer (without controlling the pH) containing 2% (w/v) polyethylene glycol (PEG20000), 3% (w/v) glycerol, 0.01% BSA, 125 mM HEPES, and 75 mM Tris-HCl was prepared as an electrophoresis buffer solution 3.

### • Electrophoresis buffer solution 4

A 75 mM Tris-HCl buffer (pH 7.5) containing 2% (w/v) polyethylene glycol (PEG20000), 3% (w/v) glycerol, and 0.01% BSA was prepared as an electrophoresis buffer solution 4.

### • DNA-labeled antibody solution (containing DNA-labeled anti-PSA antibody)

A buffer [containing 2% (w/v) polyethylene glycol (PEG20000), 0.5 mM EDTA (2Na), 3% (w/v) glycerol, 50 mM NaCl, 0.01% BSA, and 75 mM BisTris (pH 6.0)] containing a 100 nM DNA-labeled anti-PSA antibody obtained in 1) of (1) above was prepared as a DNA-labeled antibody solution.

### • Fluorescent solution

50 mM BisTris (pH 6.0) containing 30 nM HiLyte 647 and 20% (w/v) glycerol was used as a fluorescence solution. The fluorescent solution is used for adjustment such as position confirmation in a detection unit of a measurement instrument (µTAS WAKO i30).

### 3) Electrophoresis Procedure

### i) Introduction of Electrophoresis Sample A and Electrophoresis Reagent Solutions

5.4 µL of the electrophoresis sample A prepared in 1) of (2) above was dispensed into designated wells (SP wells) of a µTAS WAKO i30-exclusive microchip. Subsequently, each reagent solution prepared in 2) of (2) above was dispensed into each well of the microchip as described below.
- R2 Wells (a R2 (FLB) well and a R2 (LB) well each): 10.0 µL of electrophoresis buffer solution 2 (containing MAA and sodium chondroitin sulfate c)
- R3 Well: 10.0 µL of electrophoresis buffer solution 3
- R4 Well: 5.4 µL of electrophoresis buffer solution 4
- C1 Well: 3.0 µL of DNA-labeled antibody solution
- FD Well: 7.0 µL of fluorescent solution

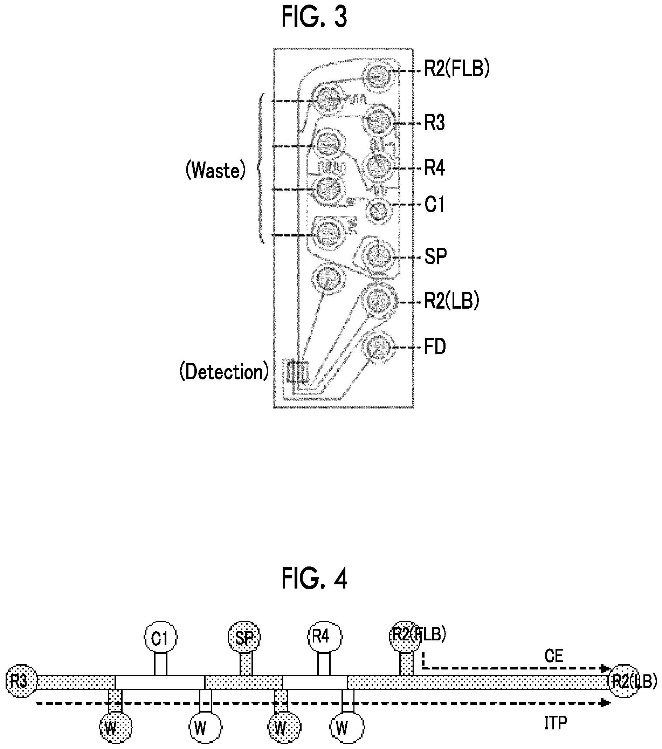

A schematic diagram of the microchip used is shown in Fig. 3.

In Fig. 3, waste wells are used as waste liquid reservoirs (drain wells) for introducing reagent solutions of the wells (R2, R3, R4, and C1) and the electrophoresis sample A into an analysis flow path.

Subsequently, a pressure of -5 psi was applied to each of the four waste wells (drain wells) for each 30 seconds to introduce the electrophoresis sample A and each reagent into the analysis flow path of the chip.

### ii) ITP (Reaction, Concentration, and Separation), and Detection

A schematic diagram of the in-chip flow path of the microchip used is shown in Fig. 4.

In Fig. 4, W indicates a waste well. The R3 well side becomes a cathode and the R2 (LB) well side becomes an anode. In addition, in Fig. 4, arrangement position of the electrophoresis sample A and each of the reagent solutions are color-coded into dotted portions and white portions (portions without dots).

After introducing the electrophoresis sample A and each of the reagent solutions into the analysis flow path of the chip, separation and detection of PSA were performed by the following method.

A voltage of 4,000 V was applied between the R3-R2(LB) wells in Fig. 4 and a DNA-labeled anti-PSA antibody in a reagent solution was brought into contact with the [fluorescence-labeled anti-free PSA antibody - r-free PSA] complex in the electrophoresis sample A at 30°C to form a complex of [fluorescence-labeled anti-free PSA antibody - r-free PSA - DNA-labeled anti-PSA antibody] which was then concentrated through isotachophoresis (ITP). The electrophoretic direction of the isotachophoresis is shown by "ITP" and a dotted line in Fig. 4.

The immunoreaction time with each labeled antibody for capturing free PSA was about 200 seconds.

The complexes formed here are specifically a complex of [fluorescence-labeled anti-free PSA antibody - rα(2,3) sugar chain free PSA - DNA-labeled anti-PSA antibody] (complex 1) and a complex of [fluorescence-labeled anti-free PSA antibody - free PSA other than rα(2,3) sugar chain free PSA - DNA-labeled anti-PSA antibody] (complex 2).

It was judged from the voltage change that the complexes were subjected to isotachophoresis up to a channel cross position of R2(FLB) and R2(LB) and passed through the channel cross position, and the negative electrode was switched from R3 to R2(FLB). Capillary gel electrophoresis (CE) was performed in the presence of MAA and sodium chondroitin sulfate c until a peak of the complex of [fluorescence-labeled anti-free PSA antibody - r-free PSA - DNA-labeled anti-PSA antibody] was further detected in a detection position (a capillary zone 2 cm downstream from the channel cross portion of R2(FLB) and R2(LB)). The position where the CE is performed and the electrophoretic direction of the electrophoresis are shown by "CE" and a dotted line in Fig. 4.

The complex 1 and the complex 2 are brought into contact with MAA to cause a reaction in the presence of sodium chondroitin sulfate c while performing the capillary gel electrophoresis in the presence of MAA and sodium chondroitin sulfate c. During the reaction, the concentration of MAA in the reaction solution is 4 mg/mL and the concentration (w/v%) of sodium chondroitin sulfate c is 0%, 2.8%, 3.4%, 3.5%, 3.6%, 3.8%, 4.1%, 4.3%, or 4.5%.

The detection was performed by measuring the fluorescence intensity of the capillary zone located at 2 cm from the channel cross position of R2(FLB) and R2(LB) through 635 nm laser excitation with PHOTODIODE (manufactured by FUJIFILM Corporation) over time.

### (3) Results

The peak area of a fraction of the complex of [fluorescence-labeled anti-free PSA antibody - rα(2,3) sugar chain free PSA - DNA-labeled anti-PSA antibody] (complex 1) reacted with MAA was obtained from an obtained electrophoretic image (electropherogram) using analysis software equipped to the i30 device.

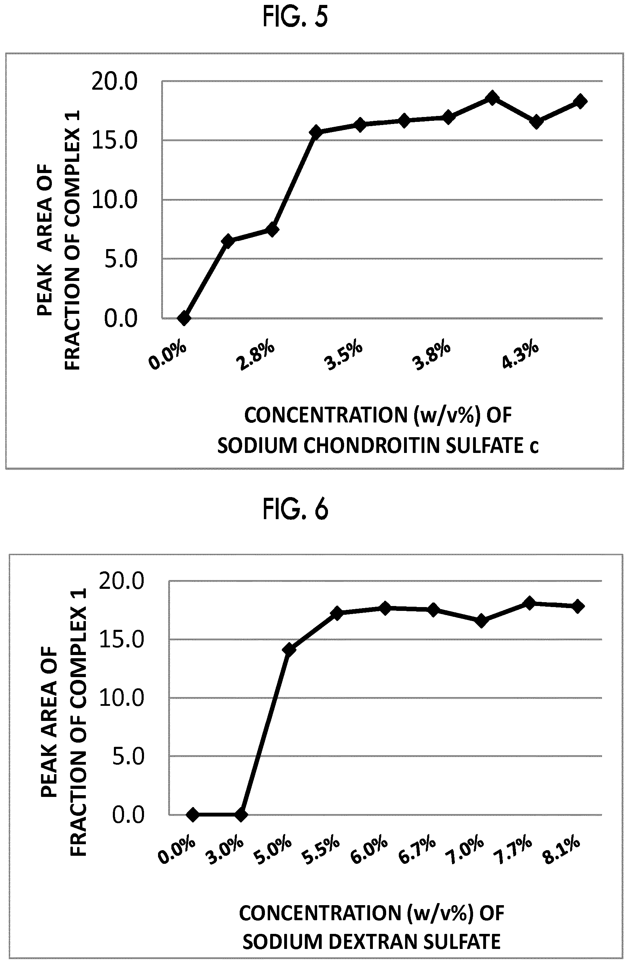

The results are shown in Fig. 5. In Fig, 5, the longitudinal axis represents a peak area of a fraction of the complex 1 and the lateral axis represents a concentration (% (w/v%)) of sodium chondroitin sulfate c during the reaction of MAA with the complex 1 and the complex 2.

As is clear from Fig. 5, the peak area of the complex 1 increased depending on the concentration of sodium chondroitin sulfate c. Accordingly, it can be seen that the amount of complex 1 is increased by performing a reaction of a sugar chain with a lectin in the presence of sodium chondroitin sulfate c compared to a case where the same reaction is performed in the absence of sodium chondroitin sulfate c.

It should be noted that since an electrophoresis reagent 2 contains polyethylene glycol (PEG8000; 5%), a substance having a sugar chain is reacted with a lectin in the presence of polyethylene glycol and in the absence of sodium chondroitin sulfate c under the condition that the concentration of sodium chondroitin sulfate c becomes "0 (w/v)%". As is clear from Fig. 5, the complex 1 could hardly be detected in the case where a substance having a sugar chain is reacted with a lectin in the presence of polyethylene glycol and in the absence of sodium chondroitin sulfate c. From the results, it can be seen that, even in a case where a substance having a sugar chain is reacted with a lectin in the absence of polysaccharides according to the present invention and in the presence of polyethylene glycol which is a high-molecular polymer, there is no effect of increasing the amount of complex.

### Example 3. Detection 2 of α(2,3) sugar chain free PSA

### (1) Preparation of Sample and Reagent Solution and Electrophoresis (Microchip Capillary Electrophoresis)

A complex of [fluorescence-labeled anti-free PSA antibody - α(2,3) sugar chain free PSA - DNA-labeled anti-PSA antibody] (complex 1) was detected through the same method as in Example 2 using the same electrophoresis reagent solutions, device, and the like as those used in Example 2 except that the following electrophoresis buffer solution 2 is used.

### • Electrophoresis buffer solution 2 (containing polysaccharides according to present invention)

A 75 mM Tris-HCl buffer (pH 7.5) containing 5.0% (w/v) polyethylene glycol (PEG8000), 3% (w/v) glycerol, 10 mM NaCl, and 0.01% BSA was prepared. MAA (manufactured by VECTOR) and sodium dextran sulfate (M.W. 6,500 to 10,000) (manufactured by Sigma), which was as polysaccharides according to the present invention, were added to and mixed with the 75 mM Tris-HCl buffer so that the final concentration (w/v) (concentration in a electrophoresis buffer solution 2) of MAA became 4 mg/mL and each final concentration (concentration in an electrophoresis buffer solution 2) of sodium dextran sulfate became 0%, 3.0%, 5.0%, 5.5%, 6.0%, 6.7%, 7.0%, 7.7%, and 8.1% to prepare each electrophoresis buffer solution 2.

It should be noted that, when the complex 1 and the complex 2 were reacted with MAA in the presence of sodium dextran sulfate, the concentration of MAA in the reaction solution is 4 mg/mL and the concentration (w/v%) of sodium dextran sulfate is 0%, 3.0%, 5.0%, 5.5%, 6.0%, 6.7%, 7.0%, 7.7%, or 8.1%.

### (2) Results

The peak area of a fraction of the complex of [fluorescence-labeled anti-free PSA antibody - α(2,3) sugar chain free PSA - DNA-labeled anti-PSA antibody] (complex 1) reacted with MAA was obtained from an obtained electrophoretic image (electropherogram) using analysis software equipped to the i30 device.

The results are shown in Fig. 6. In Fig, 6, the longitudinal axis represents a peak area of a fraction of the complex 1 and the lateral axis represents a concentration (% (w/v%)) of polysaccharides of the present invention during the reaction of MAA with the complex 1 and the complex 2.

As is clear from Fig. 6, the peak area of the complex 1 increased depending on the concentration of sodium dextran sulfate added. Accordingly, it can be seen that the amount of complex 1 is increased by performing a reaction of a sugar chain with a lectin in the presence of sodium dextran sulfate compared to a case where the same reaction is performed in the absence of sodium dextran sulfate.

It should be noted that since an electrophoresis reagent 2 contains polyethylene glycol (PEG8000; 5%), a sugar chain is reacted with a lectin in the presence of polyethylene glycol and in the absence of sodium dextran sulfate under the condition that the concentration of sodium dextran sulfate becomes "0 (w/v)%". As is clear from Fig. 6, the complex 1 could hardly be detected in the case where a substance having a sugar chain is reacted with a lectin in the presence of polyethylene glycol and in the absence of sodium dextran sulfate. From the results, it can be seen that, even in a case where a substance having a sugar chain is reacted with a lectin in the absence of polysaccharides according to the present invention and in the presence of polyethylene glycol which is a high-molecular polymer, there is no effect of increasing the amount of complex.

### Industrial Applicability

According to the method for forming a complex according to the embodiment of the present invention, the amount of complex of a substance having a sugar chain and a lectin increases.

For this reason, it is possible to perform highly sensitive measurement of the substance having a sugar chain by applying the method for forming a complex according to the embodiment of the present invention to the measurement of the substance having a sugar chain.

In addition, the method for forming a complex according to the embodiment of the present invention can be used for any reaction, detection, measurement, analysis of a sugar chain, and the like in which affinity of a lectin with a sugar chain is used. Moreover, it is possible to increase the sensitivity of the detection or the measurement and accurately analyze a sugar chain.

## Claims

1. A method for forming a complex of substance having a sugar chain and a lectin having affinity with the sugar chain of the substance having a sugar chain, the method comprising:
bringing a sample containing the substance having a sugar chain into contact with the lectin in the presence of a water-soluble polysaccharide having no N-acetylglucosamine or a water-soluble compound having a polysaccharide having no N-acetylglucosamine.

2. The method according to claim 1,
wherein the water-soluble polysaccharide having no N-acetylglucosamine or the water-soluble compound having a polysaccharide having no N-acetylglucosamine does not have a sugar chain with which the lectin has affinity.

3. The method according to claim 1,
wherein the water-soluble polysaccharide having no N-acetylglucosamine or the water-soluble compound having a polysaccharide having no N-acetylglucosamine is dextran sulfate or a salt thereof, or chondroitin sulfate c or a salt thereof.

4. The method according to claim 1, which is selected from the following (1) and (2):
wherein (1) the water-soluble polysaccharide having no N-acetylglucosamine is dextran sulfate or a salt thereof, the substance having a sugar chain is α-fetoprotein-L3 (AFP-L3), and the lectin is a lens culinaris agglutinin, and
wherein (2) the water-soluble polysaccharide having no N-acetylglucosamine or the water-soluble compound having a polysaccharide having no N-acetylglucosamine is dextran sulfate or a salt thereof, or chondroitin sulfate c or a salt thereof, the substance having a sugar chain is an α(2,3)-sugar chain free type prostate specific antigen, and the lectin is Maackia amurensis lectin.

5. An enhancer for forming a complex of a substance having a sugar chain and a lectin having affinity with the sugar chain of the substance having a sugar chain, the enhancer comprising:
a water-soluble polysaccharide having no N-acetylglucosamine or a water-soluble compound having a polysaccharide having no N-acetylglucosamine.

6. The enhancer for forming a complex according to claim 5,
wherein the water-soluble polysaccharide having no N-acetylglucosamine or the water-soluble compound having a polysaccharide having no N-acetylglucosamine does not have a sugar chain with which the lectin has affinity.

7. The enhancer for forming a complex according to claim 5,
wherein the water-soluble polysaccharide having no N-acetylglucosamine or the water-soluble compound having a polysaccharide having no N-acetylglucosamine is dextran sulfate or a salt thereof, or chondroitin sulfate c or a salt thereof.

8. The enhancer for forming a complex according to claim 5, which is selected from the following (1) and (2):
wherein (1) the water-soluble polysaccharide having no N-acetylglucosamine is dextran sulfate or a salt thereof, the substance having a sugar chain is α-fetoprotein-L3 (AFP-L3), and the lectin is a lens culinaris agglutinin, and
wherein (2) the water-soluble polysaccharide having no N-acetylglucosamine or the water-soluble compound having a polysaccharide having no N-acetylglucosamine is dextran sulfate or a salt thereof, or chondroitin sulfate c or a salt thereof, the substance having a sugar chain is an α(2,3)-sugar chain free type prostate specific antigen, and the lectin is Maackia amurensis lectin.

9. A method for measuring a substance having a sugar chain, the method comprising:
forming the complex of the substance having a sugar chain and the lectin in the method according to claim 1; and
measuring an amount of the complex.
